# EUROPEAN PATENT SPECIFICATION

(11) **EP 1 833 987 B1**
(45) Date of publication and mention of the grant of the patent: **08.08.2012**
(21) Application number: 05817755.1
(22) Date of filing: 15.12.2005
(51) Int. Cl.: G01N 33/53, G01N 33/543

(54) **A SINGLE-STEP PLATFORM FOR ON-CHIP INTEGRATION OF BIO-MOLECULES**
EIN-SCHRITT-PLATTFORM FÜR DIE ON-CHIP-INTEGRATION VON BIOMOLEKÜLEN
PLATEFORME EN UNE ÈTAPE POUR L'INTÉGRATION DES BIO-MOLECULES SUR UNE PUCE

(30) Priority: 15.12.2004 US 635937 P
(43) Date of publication of application: 19.09.2007
(73) Proprietor: YEDA RESEARCH AND DEVELOPMENT CO., LTD., 76100 Rehovot (IL)
(72) Inventor: BAR-ZIV, Roy, 84965 Omer (IL); MORPURGO, Margherita, I-35100 Padova (IT); BUXBOIM, Amnon, 30900 Zichron Ya'acov (IL); BAR-DAGAN, Maya, 42439 Natania (IL); FRYDMAN, Veronica, 76100 Rechovot (IL); ZBAIDA, David, 53520 Givataim (IL)
(74) Representative: Machtalère, Georges
(86) International application number: PCT/IL2005/001352
(87) International publication number: WO 2006/064505

(56) References cited:
- WO-A-02/20150
- US-A- 5 853 744
- US-B1- 6 444 318
- US-B2- 6 815 078
- SHIN DONG-SIK ET AL: "Protein patterning by maskless photolithography on hydrophilic polymer-grafted surface." BIOSENSORS & BIOELECTRONICS, vol. 19, no. 5, 30 December 2003 (2003-12-30), pages 485-494, XP002511389 ISSN: 0956-5663
- DILLMORE W SHANNON ET AL: "A photochemical method for patterning the immobilization of ligands and cells to self-assembled monolayers" LANGMUIR, ACS, WASHINGTON, DC, US, vol. 20, no. 17, 17 August 2004 (2004-08-17), pages 7223-7231, XP002350561 ISSN: 0743-7463
- BLAWAS A S ET AL: "Protein patterning" BIOMATERIALS, ELSEVIER SCIENCE PUBLISHERS BV., BARKING, GB, vol. 19, no. 7-9, 5 April 1998 (1998-04-05), pages 595-609, XP004120826 ISSN: 0142-9612
- SHIN D.-S. ET AL.: 'Protein Patterning by Maskless Photolithography on Hydrophilic Polymer-Grafted Surface' BIOSENSORS AND BIOELECTRONICS vol. 19, 2003, pages 485 - 494, XP002511389

### SEQUENCE LISTING

<110> Yeda Research And Development Co. Ltd.
   Bar-Ziv, Roy
   Morpurgo, Margherita
   Buxboim, Amnon
   Bar-Dagan, Mava
   Frydman, Veronica
   Zbaida, David
<120> A SINGLE-STEP PLATFORM FOR ON-CHIP INTEGRATION OF BIO-MOLECULES
<130> 30786
<160> 3
<170> Patent In version 3.3
<210> 1
   <211> 9
   <212> PRT
   <213> Artificial sequence
<220>
   <223> HA tag peptide sequence
<400> 1
   Tyr Pro Tyr Asp Val Pro Asp Tyr Ala
   1               5
<210> 2
   <211> 25
   <212> DNA
   <213> Artificial sequence
<220>
   <223> Single stand DNA oligonucleotide
<400> 2
   catgccatgg tgagcaaggg cgagg 25
<210> 3
   <211> 28
   <212> DNA
   <213> Artificial sequence
<220>
   <223> Single stand DNA oligonucleotide
<400> 3
   tcccccgggc ttgtacagct cgtccatg 28

## Description

### FIELD AND BACKGROUND OF THE INVENTION

The present invention relates to the field of microarrays and, more particularly, to novel photoactivatable compounds for efficiently forming microarrays.

Genomics and proteomics have delivered massive amounts of information and data about life's molecular components, moving the bottle neck of drug discovery downstream by providing targets and leads to companies and laboratories focused on drug discovery and improved diagnostics. For example, the sequencing of the human genome has led to the identification of approximately 30,000 genes. These 30,000 genes, in turn, can generate many-fold greater diversity in message RNA transcripts through alternate splicing reactions. Even more diversity is created through processing of the message RNA into proteins and further post-translational modifications.

Moreover, the combination of chemical processes such as alternative RNA splicing, protein processing and post-translational modifications increase the diversity of chemical entities into the millions. Further, the chemical environment of a cell is largely controlled by the proteins in the cell. Therefore, information about the abundance, modification state, and activity of the proteins in a cellular sample is extremely valuable in understanding cellular biology. All of this genotypic and phenotypic information is vital to the development of new pharmaceutical and better diagnostic tests for the treatment of disease and therefore needs to be examined.

To this end, a multitude of technologies have been designed to gather biological information on a faster and faster scale. For example, robotics and miniaturization technologies can lead to advances in the rate at which information on complex samples is generated. High-throughput screening technologies permit routine analysis of tens of thousands of samples. More specifically, microfluidics and DNA array technologies permit information from a single sample to be gathered in a massively parallel manner. For example, DNA array chips can simultaneously measure the quantity of more than 10,000 different RNA molecules in a sample in a single experiment.

Thus, DNA and proteome array and microarray technologies make it possible to efficiently analyze gene expression and function. These technologies can also validate and optimize drug targets; evaluate a potential drug's mode of action or potential toxic side effects; monitor the genetic stability of cell lines used in research; identify previously undetected phenotypes resulting from genetic changes in cell lines; and compare normal and diseased cells for drug discovery, diagnostics and toxicogenomics, in a single or high-throughput format, thereby decreasing the time required to identify or validate a particular diagnostic technique or therapeutic compound. Therefore, microarray technology has evolved to become an important tool.

Microarrays derive their name from the small (e.g., about 20-750 micron) size of the analysis sites typically arranged in a two-dimensional matrix of probe elements on the surface of a supporting substrate. The range of microarray samples is varied.

In the majority of current applications, each probe element comprises numerous identical oligonucleotide (DNA) "probe" molecules. These probes are fixed to the substrate surface and may hybridize with complementary oligonucleotide "targets" from a sample. Typically, a label (e.g., fluorescent molecule) is either attached to the target prior to the hybridization step, or to the probe/target complex subsequent to hybridization. The microarrays are then observed for the presence of detectable labels (fluorescence imaging). The presence of a label in the area encompassing a particular probe element indicates that a sequence complementary to the characteristic sequence of that element was in the analyte.

Microarrays are known in the art and are commercially available from a number of sources. Microarrays have been used for a number of analytical purposes, typically in the biological sciences. An array is essentially a two-dimensional sheet where different portions or cells of the sheet have different bimolecular elements, such as, nucleic acids or peptides, bound thereto. Microarrays are similar in principle to other solid phase arrays except that assays involving such microarrays are performed on a smaller scale, allowing many assays to be performed in parallel. For example, the reactive bimolecular can be bound to a chip.

Biochemical molecules on microarrays have been synthesized directly at or on a particular cell on the microarray, or preformed molecules have been attached to particular cells of the microarray by chemical coupling, adsorption or other means. The number of different cells and therefore the number of different biochemical molecules being tested simultaneously on one or more microarrays can range into the thousands. Commercial microarray plate readers typically measure fluorescence in each cell and can provide data on thousands of reactions simultaneously thereby saving time and labor. A representative example of the dozens of patents in this field is U.S. Pat. No. 5,545,531.

Two dimensional arrays of macromolecules can be made either by depositing small aliquots on flat surfaces under conditions which allow the macromolecules to bind or be bound to the surface, or the macromolecules may by synthesized on the surface using light-activated or other reactions. Other methods include using printing techniques to produce such arrays. Some methods for producing arrays have been described in "Gene-Expression Micro-Arrays: A New Tool for Genomics", Shalon, D., in Functional Genomics: Drug Discovery from Gene to Screen, IBC Library Series, Gilbert, S. R. & Savage, L. M., eds., International Business Communications, Inc., Southboro, Mass., 1997, pp 2.3.1.-2.3.8; "DNA Probe Arrays: Accessing Genetic Diversity", Lipshutz, R. J., in Functional Genomics: Drug Discovery from Gene to Screen, IBC Library Series, Gilbert, S. R. & Savage, L. M., eds., International Business Communications, Inc., Southboro, Mass., 1997, pp 2.4.1.-2.4.16; "Applications of High-Throughput Cloning of Secreted Proteins and High-Density Oligonucleotide Arrays to Functional Genomics", Langer-Safer, P. R., in Functional Genomics: Drug Discovery from Gene to Screen, IBC Library Series, Gilbert, S. R. & Savage, L. M., International Business Communications, Inc., Southboro, Mass., 1997, pp 2.5.13; Jordan, B. R., "Large-scale expression measurement by hybridization methods: from high-densities to "DNA chips"", J. Biochem. (Tokyo) 124: 251-8, 1998; Hacia, J. G., Brody, L. C. & Collins, F. S., "Applications of DNA chips for genomic analysis", Mol. Psychiatry 3: 483-92, 1998; and Southern, E. M., "DNA chips: Analyzing sequence by hybridization to oligonucleotides on a large scale", Trends in Genetics 12:110-5, 1996.

The advancement of bio-electronic devices with on-chip integrated networks of biological macromolecules such as DNA and proteins relies on a compatible interface between silicon-based materials and biological aqueous solutions in contact. Such a molecular interface has to meet a number of stringent requirements. It must form a smooth and stable film on the solid surface and allow for specific and sequential localization of various molecules at submicron resolution, possibly in combination with an integrated electronic pattern. Importantly, integrated proteins and DNA must retain their functionality as well as accessibility to interact with other molecules. While a variety of interfaces have been developed, there is yet no single molecular interface that meets *all* of the above requirements.

Presently, several strategies are used for immobilization of DNA and proteins. The simplest one is to spot the molecules onto a surface to which they have some *non-specific* physical affinity. For example, poly-lysine-coated surfaces bind DNA by electrostatic interaction [1]. The main shortcoming of this method is irreproducibility and reduced signal-to-noise ratio due to the limited control over the orientation and density of adsorbed molecules. As a result, there is considerable loss of functionality on the surface. Next-generation biochips use covalent or biospecific immobilization. The common approach is to build subsequent reactive layers by a multi-step procedure. Ordered and dense monolayers can be achieved by control of layer construction. The first layer, most commonly based on an organosilane compound, serves as anchorage to the inorganic substrate while the top layer provides the reactive groups for immobilization of the desired biological function [2, 3]. Alternatively, complete molecules can be synthesized in solution, and then grafted on a surface in a single step, thereby circumventing multiple on-surface reactions, which might lead to condensation, random orientation and multi-layering as reported for organosilanes [4-6]. Classic bio-conjugation chemistries are used for covalent immobilization, among which most common is through the formation of peptide bonds via active ester intermediates [7-10]. Alternatively, non-covalent yet high-affinity interactions are exploited, among which are the incorporation on the surface of nitrilotriacetic acid (NTA) [11, 12] termini for interaction with hexahistidine tags, or the use of the avidin-biotin pair [13].

Biocompatibility of grafted layers is often enhanced by incorporating an intermediate layer of polyethylenglycol (PEG), a polymer known for its resistance to nonspecific adsorption of proteins [14-20]. Examples are biotin-functionalized PEG grafted on PEI-modified silicon wafers [21] and PLL-g-PEG-biotin copolymers adsorbed on negatively charged surfaces [22]. Protein resistance of a grafted PEG layer results from a combination of Van-der-Waals attractions and stearic repulsion of the grafted PEG chains as it is compressed by an adsorbed protein [15, 16].

Inspired by microelectronics, light-directed immobilization, where a reactive group is immobilized on the surface in a chemically protected form and is de-protected prior to coupling by UV light, has also been developed. Photo-masks have thus been used to generate a desired surface pattern and allow sequential or parallel *in situ* synthesis of DNA oligomers and short peptides [23]. Extension of the initial approach includes: a hetero-bi-functional reagent that binds carbon-containing supports upon UV-irradiation and interacts with aminoalkyls or mercaptoalkyls in biomolecules [24]; a photoactive PEG-organosilane where the PEG is removed with UV light, exposing aldehydes to bind protein amines [25]; stepwise *in situ* synthesis of a photoactive PEG-organosilane with protected hydroxyl groups for reacting with nucleotides via phosphoramidite chemistry [26]; and a stepwise construction of aminopropyl-triethoxy-silane (APTS) layer and copolymer containing short PEG with photocaged amine terminus which becomes available upon UV radiation [3]. U.S. Pat. No 6,444,318 describes the coupling of linoleamide polyethylene glycol to a silanized substrate by photoactivation.

The non-lithographic methods described above typically involve complicated successive *in situ* step-wise attachments and are therefore disadvantageous.

The presently known light-directed immobilization methods typically involve multi-step reactions and therefore require quality control of each step, and often suffer from uncontrolled layer deposition and reduced signal-to-noise ratio due to errors accumulated at each step. Reduced signal-to-noise ratio directly limits the possibility for obtaining precise and miniaturized patterns on the surface. Furthermore, multi-step approaches necessitate surface chemistry expertise.

There is thus a widely recognized need for, and it would be highly advantageous to have, a novel technology for integrating biomolecules to microarrays, devoid of the above limitations.

### SUMMARY OF THE INVENTION

In a search for a novel technology for immobilizing various substances and particularly biological moieties and thus for producing microarrays of these substances, the present inventors have designed and successfully prepared and utilized a novel compound, in which three successfully proven approaches are combined: self-assembly of organosilanes on silicon-dioxides; biocompatibility of ethylene-glycol polymers; and *in situ* bond formation by light-direct lithographical de-protection. This novel technology is simple and inexpensive and can serve as an improved platform for a wide variety of microarrays applications.

The various aspects of the present invention are summarized in claims 1-34.

The present invention successfully addresses the shortcomings of the presently known configurations by providing a simple, cost-effective and efficient technology for use in the formation of microarrays while minimizing non-specific absorption thereto.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

As used in this application, the singular form "a," "an," and "the" include plural references unless the context clearly dictates otherwise. For example, the term "an agent" includes a plurality of agents, including mixtures thereof.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used herein throughout, the term "comprising" means that other steps and ingredients that do not affect the final result can be added. This term encompasses the terms "consisting of" and "consisting essentially of'.

The phrase "consisting essentially of" means that the composition or method may include additional ingredients and/or steps, but only if the additional ingredients and/or steps do not materially alter the basic and novel characteristics of the claimed composition or method.

The term "method" or "process" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein, the term "amine" describes both a -NR'R" group and a -NR'-group, wherein R' and R" are each independently hydrogen, alkyl, cycloalkyl, aryl, as these terms are defined herein.

The amine group can therefore be a primary amine, where both R' and R" are hydrogen, a secondary amine, where R' is hydrogen and R" is alkyl, cycloalkyl or aryl, or a tertiary amine, where each of R' and R" is independently alkyl, cycloalkyl or aryl.

Alternatively, R' and R" can each independently be hydroxyalkyl, trihaloalkyl, cycloalkyl, alkenyl, alkynyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, carbonyl, C-carboxylate, O-carboxylate, N-thiocarbamate, O-thiocarbamate, urea, thiourea, N-carbamate, O-carbamate, C-amide, N-amide, guanyl, guanidine and hydrazine.

The term "amine" is used herein to describe a -NR'R" group in cases where the amine is an end group, as defined hereinunder, and is used herein to describe a - NR'- group in cases where the amine is a linking group.

Herein throughout, the phrase "end group" describes a group (a substituent) that is attached to another moiety in the compound via one atom thereof.

The phrase "linking group" describes a group (a substituent) that is attached to another moiety in the compound via two or more atoms thereof.

The term "alkyl" describes a saturated aliphatic hydrocarbon including straight chain and branched chain groups. Preferably, the alkyl group has 1 to 20 carbon atoms. Whenever a numerical range; *e.g*., "1-20", is stated herein, it implies that the group, in this case the alkyl group, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or unsubstituted. Substituted alkyl may have one or more substituents, whereby each substituent group can independently be, for example, hydroxyalkyl, trihaloalkyl, cycloalkyl, alkenyl, alkynyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, C-carboxylate, O-carboxylate, N-thiocarbamate, O-thiocarbamate, urea, thiourea, N-carbamate, O-carbamate, C-amide, N-amide, guanyl, guanidine and hydrazine.

The alkyl group can be an end group, as this phrase is defined hereinabove, wherein it is attached to a single adjacent atom, or a linking group, as this phrase is defined hereinabove, connecting another moiety at each end thereof.

The term "cycloalkyl" describes an all-carbon monocyclic or fused ring (*i.e.,* rings which share an adjacent pair of carbon atoms) group where one or more of the rings does not have a completely conjugated pi-electron system. The cycloalkyl group may be substituted or unsubstituted. Substituted cycloalkyl may have one or more substituents, whereby each substituent group can independently be, for example, hydroxyalkyl, trihaloalkyl, cycloalkyl, alkenyl, alkynyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, C-carboxylate, O-carboxylate, N-thiocarbamate, O-thiocarbamate, urea, thiourea, N-carbamate, O-carbamate, C-amide, N-amide, guanyl, guanidine and hydrazine. The cycloalkyl group can be an end group, as this phrase is defined hereinabove, wherein it is attached to a single adjacent atom, or a linking group, as this phrase is defined hereinabove, connecting two or more moieties at two or more positions thereof.

The term "aryl" describes an all-carbon monocyclic or fused-ring polycyclic (*i.e*., rings which share adjacent pairs of carbon atoms) groups having a completely conjugated pi-electron system. The aryl group may be substituted or unsubstituted. Substituted aryl may have one or more substituents, whereby each substituent group can independently be, for example, hydroxyalkyl, trihaloalkyl, cycloalkyl, alkenyl, alkynyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, C-carboxylate, O-carboxylate, N-thiocarbamate, O-thiocarbamate, urea, thiourea, N-carbamate, O-carbamate, C-amide, N-amide, guanyl, guanidine and hydrazine. The alkyl group can be an end group, as this term is defined hereinabove, wherein it is attached to a single adjacent atom, or a linking group, as this term is defined hereinabove, connecting two or more moieties at two or more positions thereof.

The term "halide" or "halo" describes fluorine, chlorine, bromine or iodine.

The term "haloalkyl" describes an alkyl group as defined above, further substituted by one or more halide.

The term "sulfate" describes a -O-S(=O)₂-OR' end group, or an -O-S(=O)₂-O- linking group, as these phrases are defined hereinabove, where R' is as defined hereinabove. This term further encompasses thiosulfates.

The term "thiosulfate" describes a -O-S(=S)(=O)-OR' end group or a -O-S(=S)(=O)-O- linking group, as these phrases are defined hereinabove, where R' is as defined hereinabove.

The term "sulfonate" describes a -S(=O)₂-R' end group or an -S(=O)₂- linking group, as these phrases are defined hereinabove, where R' is as defined herein.

The term "sulfonamide" describes a -S(=O)₂-NR'R" end group or a -S(=O)₂-NR'- linking group, as these phrases are defined hereinabove, with R' and R" as defined herein. This term encompasses the terms N-sulfonamide and S-sulfonamide.

The term "N-sulfonamide" describes an R'S(=O)₂-NR"- end group or a -S(=O)₂-NR'- linking group, as these phrases are defined hereinabove, where R' and R" are as defined herein.

The term "S-sulfonamide" describes an -S(=O)₂-NR'R"- end group or a -S(=O)₂-NR'- linking group, as these phrases are defined hereinabove, where R' and R" are as defined herein.

The term "phosphonate" describes a -P(=O)(OR')(OR") end group or a -P(=O)(OR')(O)- linking group, as these phrases are defined hereinabove, with R' and R" as defined herein.

The term "phosphate" describes an -O-P(=O)₂(OR') end group or a -O-P(=O)₂(O)- linking group, as these phrases are defined hereinabove, with R' as defined herein. This term further encompasses the term thiophosphonate.

The term "thiophosphate" describes an -O-P(=O)(=S)(OR') end group or a -O-P(=O)(=S)(O)- linking group, as these phrases are defined hereinabove, with R' as defined herein.

The term "carbonyl" or "carbonylate" as used herein, describes a -C(=O)-R' end group or a -C(=O)- linking group, as these phrases are defined hereinabove, with R' as defined herein. Alternatively, R' can be halide, or any other reactive derivative. This term encompasses the term "thiocarbonyl".

The term "thiocarbonyl " as used herein, describes a -C(=S)-R' end group or a -C(=S)- linking group, as these phrases are defined hereinabove, with R' as defined herein.

The term "hydroxyl" describes a -OH group.

The term "alkoxy" describes both an -O-alkyl and an -O-cycloalkyl group, as defined herein.

The term "aryloxy" describes both an -O-aryl and an -O-heteroaryl group, as defined herein.

The term "thiohydroxy" describes a -SH group.

The term "thioalkoxy" describes both a -S-alkyl group, and a -S-cycloalkyl group, as defined herein.

The term "thioaryloxy" describes both a -S-aryl and a -S-heteroaryl group, as defined herein.

The term "cyano" describes a -C≡N group.

The term "isocyanate" describes an -N=C=O group.

The term "nitro" describes an -NO₂ group.

The term "azo" describes an -N=NR' end group or an -N=N- linking group, as these phrases are defined hereinabove, with R' as defined hereinabove.

The term "carboxylate" describes a -C(=O)-OR' end group or a -C(=O)-O-linking group, as these phrases are defined hereinabove, where R' is as defined herein. This term encompasses the terms O-carboxylate, C-thiocarboxylate, and O-thiocarboxylate, as well as various derivatives thereof including, but not limited to, N-hydroxysuccinimide esters, N-hydroxybenztriazole esters, acid halides, acyl imidazoles, thioesters, p-nitrophenyl esters, alkyl, alkenyl, alkynyl and aromatic esters.

The term "carbamate" describes an R"OC(=O)-NR'- end group or a -OC(=O)-NR'- linking group, as these phrases are defined hereinabove, with R' and R" as defined herein. This term encompasses the terms O-carbamate, thiocarbamate and include various derivatives thereof including, but not limited to, N-hydroxysuccinimide esters, N-hydroxybenztriazole esters, acid halides, acyl imidazoles, thioesters, p-nitrophenyl esters, alkyl, alkenyl, alkynyl and aromatic esters.

The term "amide" describes a -C(=O)-NR'R" end group or a -C(=O)-NR'-linking group, as these phrases are defined hereinabove, where R' and R" are as defined herein. This term encompasses the term N-amide.

The term "N-amide" describes a R'C(=O)-NR"- end group or a R'C(=O)-N-linking group, as these phrases are defined hereinabove, where R' and R" are as defined herein.

The term "hydrazine" describes a -NR'-NR"R"' end group or a -NR'-NR"-linking group, as these phrases are defined hereinabove, with R', R", and R'" as defined herein.

The term "heteroaryl" describes a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furan, thiophene, imidazole, oxazole, thiazole, pyrazole, pyridine, pyrimidine, quinoline, isoquinoline and purine. The heteroaryl group may be substituted or unsubstituted. Substituted heteroaryl may have one or more substituents, whereby each substituent group can independently be, for example, hydroxyalkyl, trihaloalkyl, cycloalkyl, alkenyl, alkynyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, C-carboxylate, O-carboxylate, N-thiocarbamate, O-thiocarbamate, urea, thiourea, O-carbamate, N-carbamate, C-amide, N-amide, guanyl, guanidine and hydrazine. The heteroaryl group can be an end group, as this phrase is defined hereinabove, where it is attached to a single adjacent atom, or a linking group, as this phrase is defined hereinabove, connecting two or more moieties at two or more positions thereof. Representative examples are pyridine, pyrrole, oxazole, indole, purine and the like.

The term "heteroalicyclic" describes a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or unsubstituted. Substituted heteroalicyclic may have one or more substituents, whereby each substituent group can independently be, for example, hydroxyalkyl, trihaloalkyl, cycloalkyl, alkenyl, alkynyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, C-carboxylate, O-carboxylate, N-thiocarbamate, O-thiocarbamate, urea, thiourea, O-carbamate, N-carbamate, C-amide, N-amide, guanyl, guanidine and hydrazine. The heteroalicyclic group can be an end group, as this phrase is defined hereinabove, where it is attached to a single adjacent atom, or a linking group, as this phrase is defined hereinabove, connecting two or more moieties at two or more positions thereof. Representative examples are piperidine, piperazine, tetrahydrofurane, tetrahydropyrane, morpholino and the like.

The term "ester" describes a moiety containing a carboxylate group, as defined herein.

An "alkenyl" group describes an alkyl group which consists of at least two carbon atoms and at least one carbon-carbon double bond.

An "alkynyl" group describes an alkyl group which consists of at least two carbon atoms and at least one carbon-carbon triple bond.

A "dienophile" group describes a group which comprises at least two conjugated double-double boned.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

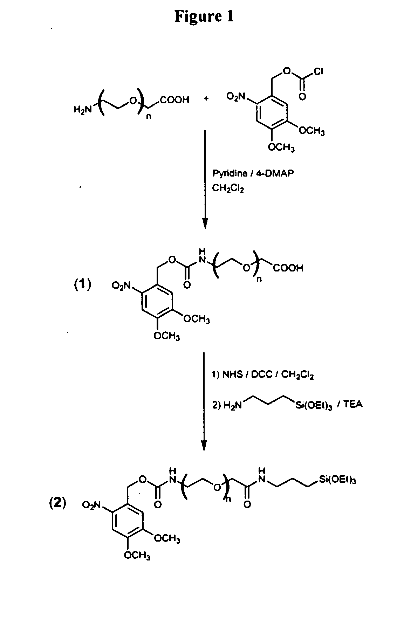

FIG. 1 is a scheme presenting a synthetic pathway of an exemplary compound according to the present invention ("daisy", Compound **2**), which upon contacting a solid substrate forms a monolayer terminating with a photoactivatable group;

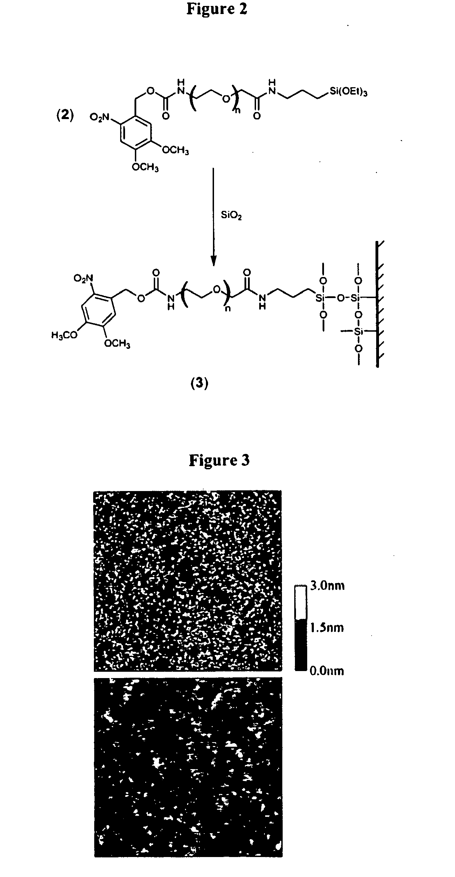

FIG. 2 is a scheme presenting a synthetic pathway of an exemplary solid substrate according to a preferred embodiment of the present invention (Compound **3**), which has Compound 2 applied thereon;
FIG. 3 presents AFM images of (1µm)² areas of a layer of SiO₂ 100 nm thick deposited on silicon wafers, before (top) and after (bottom) incubation with Compound **2**;

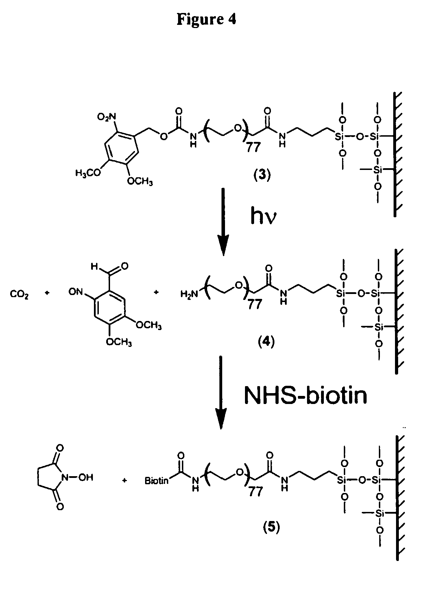

FIG. 4 is a scheme presenting a synthetic pathway of an exemplary functionalized solid support according to a preferred embodiment of the present invention, in which exposing Compound **3** to 365nm UV light through a mask generates a reactive group (Compound **4**), which thereafter forms an amide bond with *n*-hydroxysuccinimide-biotin so as to produce a biotinylated solid support (Compound **5**);

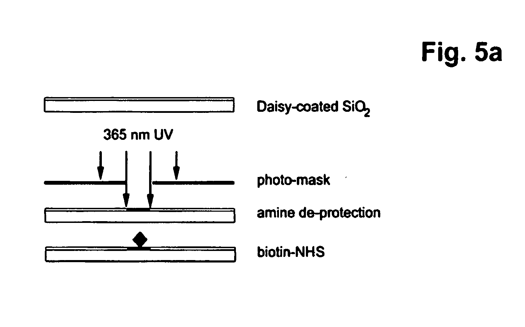

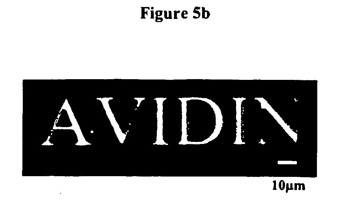

FIGs. 5a-b depict light directed localization of biomolecules to a Daisy film (e.g., compound **3**) of the present invention. Figure 5a schematically illustrates photolithography of proteins and genes. A Daisy monolayer (Daisy-coated SiO₂) is exposed to 365 nm UV light through a mask to thereby deprotect amines for amide bond formation. N-hydroxysuccinimide-biotin (i.e., mediating moiety) is then bound as a localization signal pattern for the avidin protein. Figure 5b presents an image of a 2D pattern of avidin protein labeled with fluorescein, generated with a printed 'AVIDIN' photo-mask placed at the field stop of a microscope applied during irradiation of Compound **3**, followed by biotinylation of the irradiated substrate and reaction with fluorescein-tagged avidin;

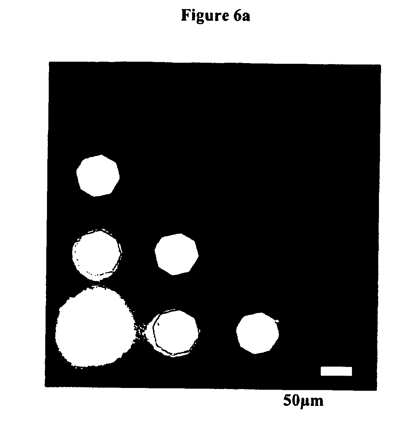

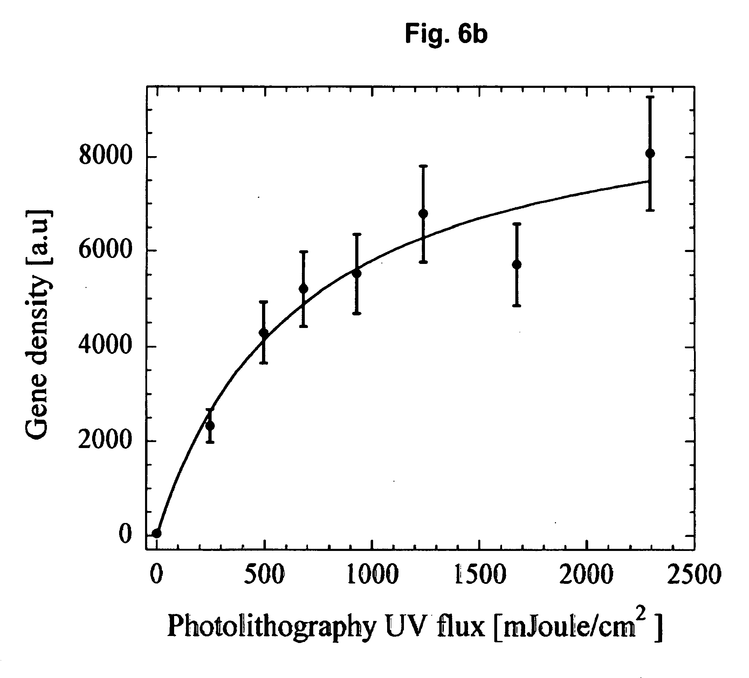

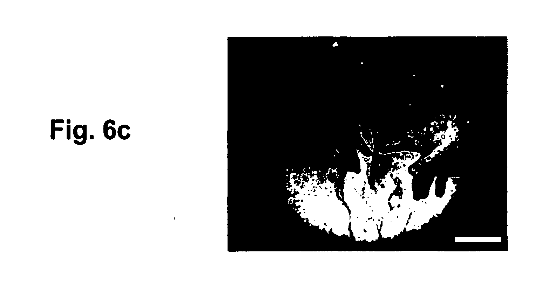

FIGs. 6a-c present control of molecular density by photolithography. Figure 6a is an image of a 2D pattern of avidin protein labeled with fluorescein, generated with an octagonal mask applied at varying duration (vertical axis up to down: 5 sec, 30 sec, 2 min and 10 min) and UV light intensity (horizontal axis (right to left): 0.44 mW/cm², 4.4 mW/cm², 18.7 mW/cm² and 70 mW/cm²) during irradiation of Compound **3**, followed by biotinylation of the irradiated substrate and reaction with fluorescein-tagged avidin (Scale bar: 50µm); Figure 6b shows a signal-to-noise ratio (S/N) for patterning DNA molecules on Daisy-coated chips. Figure 6c is a photolithography generated with Michelangelo's 'Creation of Adam' photo-mask placed at the field stop of a microscope applied during irradiation of Daisy-coated glass (Compound **3**), followed by biotinylation of the irradiated substrate and reaction with fluorescein-tagged avidin coupled to biotinylated double-stranded DNA, 2000 base pairs long. Scale bars: 50 µm;

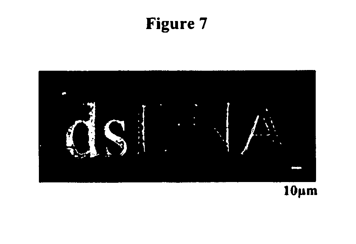

FIG. 7 presents an image of a 2D pattern of biotinilated double-stranded DNA, 2000 base pairs long, tagged with cy3 dye, which was reacted with neutra-avidin and integrated onto a 2D-patterned biotinylated solid substrate according to a preferred embodiment of the present invention, generated with a printed 'dsDNA' photo-mask placed at the field stop of a microscope applied during irradiation of Compound **3**;

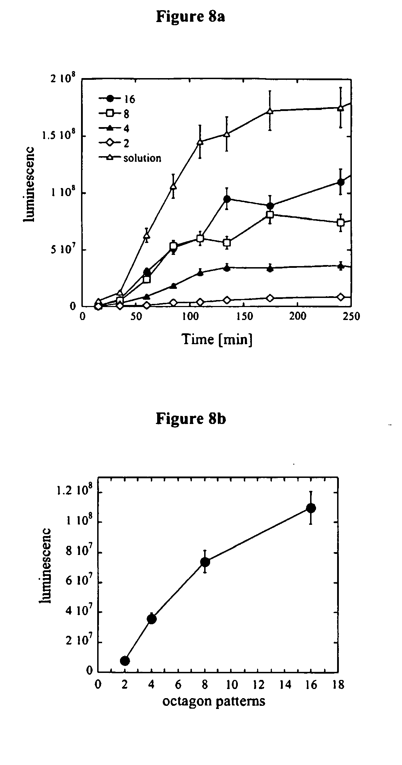

FIGs. 8a-b present comparative plots demonstrating the cell-free transcription/translation reaction and the luciferase protein synthesis as a function of time (Figure 8a) and as a function of the number of patterned DNA octagons (Figure 8b) assayed with exemplary microarrays according to the present invention to which transcriptional units encoding the firefly *luciferase* reporter gene under T7 promoter were integrated, using a ∼50x50µm² octagon pattern, whereby each microarray had increasing number of DNA octagons 2, 4, 8 and 16;

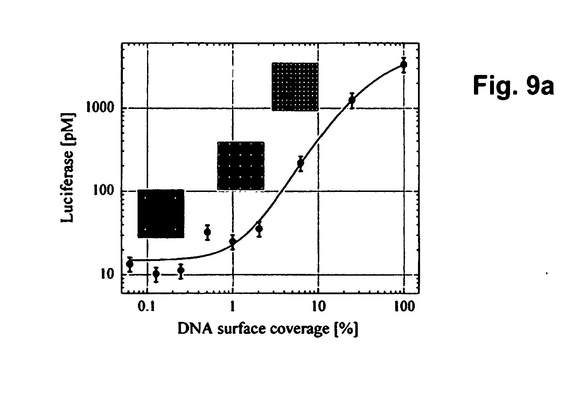

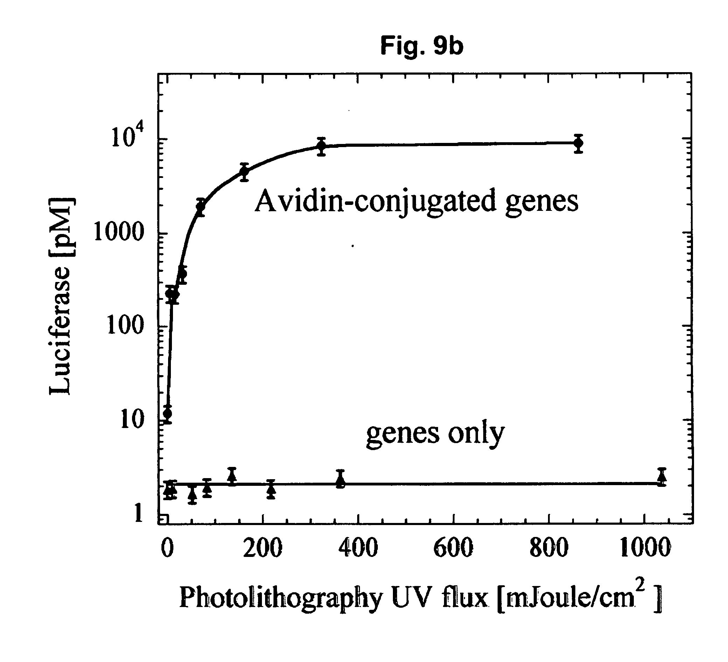

FIGs. 9a-b present the effect of DNA surface coverage and density on reporter gene firefly *luciferase in vitro* transcription/translation yield. Figure 9a - A single chip, 18x18 mm² was divided into nine regions exposed to the same UV flux, using contact-mode lithography with a mask made of 10x10 µm² squares at varying distances, from 0 to 400microns, thus covering surface coverage of 0.06% to 100%, extended over macroscopic surface, 3mm in diameter, in each region. Cell extract protein biosynthesis reactions (10 µl) were incubated with each region and Luciferase protein yield was measured after 4-5 hrs. Insets demonstrate the unit of the mask used to pattern. Figure 9b - A single chip was divided into nine regions as in Figure 9a, each exposed uniformly at varying UV flux. Genes were incubated on the chip with (top curve, circles) and without avidin (lower curve, triangles) conjugation for subsequent measurements of Luciferase protein yield as a function of gene density.

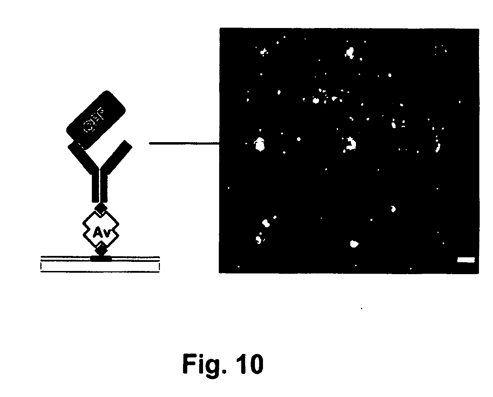

FIG. 10 present the ability of surface immobilized antibodies generated according to the teachings of the present invention, to bind a cognate antigen. Avidin was patterned using an array of 10x10µm² squares 50µm apart on a silicon dioxide wafer (100nm SiO₂ thermally grown on Silicon <100>). A biotinylated antibody to the HA tag was attached to the avidin patterned surface. C-terminus EGFP-HA fusion protein was synthesized using a cell-free expression system from its plasmid in solution. The cell-extract reaction was then incubated with the patterned antibody chip and the excess protein debris was thoroughly washed, revealing a pattern of EGFP-HA protein. Scale bar is 20µm.

### DESCRIPTION OF THE PREFERRED EMBODIMENTS

The present invention is of novel tri-functional compounds which can be used for attaching molecules to a solid substrate and thus for forming arrays. Specifically, the present invention is of novel compounds which are designed so as to form self-assembled monolayers on a solid substrate, each monolayer terminating with a photoactivatable group, such that using a common light-directed lithography, a variety of moieties can be selectively attached thereto, to thereby integrate various moieties on the solid substrate. These novel compounds can thus be used for forming arrays, integrating any screenable moiety by selecting a suitable photoactivatable group. The arrays, formed by the technology of the present invention can be used, for example, for biological and chemical syntheses, screening methods and any other application in which arrays and microarrays are beneficial.

The principles and operation of the compounds, the processes and the arrays according to the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

The growing interest in active chips [30-33] and devices (collectively referred to herein as arrays) with integrated networks of diverse moieties requires utility by interdisciplinary laboratories. In a search for a simplified integration of moieties on a solid substrate, which is further devoid of the limitations associates with the presently known techniques (as detailed hereinabove), the present inventors have designed and successfully prepared and utilized novel compounds in which three successfully proven approaches are integrated: self-assembly of organosilanes on silicon-dioxides; biocompatibility of ethylene-glycol polymers; and *in situ* bond formation by light-direct lithographical de-protection. This novel technology is simple and inexpensive and can serve as an improved platform for a wide variety of microarrays applications.

Thus, while conceiving the present invention, it was envisioned that using a polymer, which is characterized by minimized interaction with e.g., biological moieties such as proteins and nucleic acids, and further by capability of forming monolayers on a solid substrate, and attaching thereto a moiety that is capable of self-assembling on a solid substrate at one end, and a moiety that can be activated by irradiation and thus selectively generate a reactive group for binding a desired moiety to the substrate at the other end, would result in a compound that can be attached to a solid substrate in a simple, one-step reaction and can thus be beneficially used for integrating a plethora of moieties to solid substrates, while minimizing undesired absorptions.

To that end, polyethylene glycol (PEG), a polymer known for its resistance to nonspecific adsorption of proteins [14-20], has been selected as polymer. As is detailed in the Examples section that follows, a α,ω-difunctional PEG was used for attaching a reactive silyl group (triethoxysilane) at the α position thereof and a photoactivatable group (a carbamate derivative of 6-nitronertratyl chloroformate) at the ω position thereof, in a simple two-steps synthesis. As is further demonstrated in the Examples section that follows, when contacted with a silica-coated silicon substrate, this compound spontaneously forms a film of monolayers on the substrate, whereby upon irradiation, preferably using a photo mask, reactive groups are generated, in a pre-selected pattern, and thus can bind any desired moiety, at this pre-selected pattern. As is further demonstrated in the Examples section, using the compound of the present invention, non-specific absorption of the integrated moieties is substantially minimized. Thus, silica substrates to which specific genes were integrated using the compounds of the present invention were successfully utilized for cell-free transcription/translation protein synthesis.

Hence, each compound according to the present invention comprises a functionalized group capable of binding to a solid substrate , said functionalized group comprising at least one reactive silyl group; a photoactivatable group capable of generating a reactive group upon exposure to light; and a polyethylene glycol (PEG) polymer capable of forming a monolayer on a solid substrate, whereby the functionalized group and the photoactivatable group are attached to the polymer ; said polyethylene glycol having a molecular weight that ranges from 2000 grams/mol to 5000 grams/mol. The reactive group generated upon exposure to light serves for attaching a screenable moiety to the solid substrate. The compound is capable of forming on said solid substrate a monocyclyl terminating with said photoactivatable group, upon contacting said solid substrate in a one-step reaction.

According to a preferred embodiment of the present invention, the compounds can be represented by the general formula I below:

X- L-Y Formula I

wherein X is the functionalized group comprising said as least one reactive silyl group and being capable of binding to a solid substrate; L is said PEG polymer capable of forming a monolayer onto said solid substrate; and Y is said photoactivatable group capable of generating said reactive group upon exposure to light

The functionalized group is preferably selected such that it binds to the solid substrate by reacting with at least one functional group present on a surface of a solid substrate.

Preferred functionalized groups according to the present invention comprise one or more reactive silyl group(s).

As used herein, the phrase "reactive silyl group" describes a residue of a compound comprising at least one silicon atom and at least one reactive group, such as an alkoxy or halide, such that the silyl group is capable of reacting with a functional group, for example on a surface of a substrate, to form a covalent bond with the surface. For example, the reactive silyl group can react with the surface of a silica substrate comprising surface Si--OH groups to create siloxane bonds between the compound and the silica substrate.

Exemplary reactive silyl groups that are usable in the context of the present invention include, without limitation, trialkoxysilanes, alkyldialkoxysilanes, alkoxydialkylsilanes, trihalosilanes, alkyldihalosilanes and dialkylhalosilanes. Such reactive groups are easily reacted when contacted with free hydroxyl groups on a surface of solid surfaces and particularly with such hydroxyl groups on a silica surface.

Herein, the terms "silica" and "SiO₂" are used interchangeably.

In a preferred embodiment of the present invention the reactive silyl group is trialkoxysilane such as, for example, trimethoxysilane, triethoxysilane, tripopyloxysilane and the like.

The functionalized group according to the present invention may further include a chemical moiety that is terminated with the reactive silyl group. Such a chemical moiety can comprises, for example, alkyl, alkenyl, aryl, cycloalkyl and derivatives thereof, as these terms are defined herein.

Preferably, the functionalized group comprises an alkyl terminating with a trialkoxysilane.

As discussed hereinabove, the polymer is selected so as to form a monolayer on the substrate.

The polymer according to the present invention comprises polyethylene glycol (PEG). As described hereinabove, PEG is characterized by resistance to nonspecific absorptions of biomolecules and is therefore beneficial for use in some contexts of the present invention. In addition, when self-assembled on a solid substrate, PEG chains typically interact therebetween via hydrogen bonds, so as to produced a well-ordered monolayerd film.

The polyethylene glycol residue in the compounds of the present invention is derived from PEGs having a molecular weight that ranges from 2000 grams/mol to 5000 grams/mol. Such PEGs allow the productions of a monolayered film when deposited on a solid surface in the presence of a functionalized group, as described hereinabove.

The polyethylene glycol residue may be substituted or unsubstituted. PEG can be described by the general Formula II below:

**-(CR¹R²CR³R⁴O)n-** Formula II

wherein n is an integer from 10 to 200; and R¹, R², R³ and R⁴ are each independently selected from the group consisting of hydrogen, alkyl, cycloalkyl, aryl, alkenyl alkynyl, alkoxy, thioalkoxy, aryloxy and thioaryloxy.

In a preferred embodiment, the PEG is unsubstituted such that R¹, R², R³ and R⁴ are each hydrogen.

In a preferred embodiment, the PEG residue is a medium-sized residue such that n is an integer from 60 to 100.

The polymer is preferably attached to the functionalized group described above via a linking moiety.

Exemplary linking moieties include, without limitation, oxygen, sulfur, amine, amide, carboxylate, carbamate, sulphonate, sulphonamide, phosphate, hydrazine, hydrazide, as these terms are defined herein and derivatives thereof.

In a representative example the linking moiety is an amide, formed between a carboxylic end group of the polymer and an amine end group of the functionalized moiety, as is detailed hereinunder.

The compounds of the present invention, by comprising the functionalized group and the polymer described hereinabove, readily form self-assembled monolayers when contacted with a solid substrate, in a one-step, simple to perform, reaction.

As the polymer residue in the compounds of the present invention further has a photoactivatable group attached thereto, each of the formed monolayers has a photoactivatable group attached thereto.

As used herein, the phrase "photoactivatable group" describes a group that is rendered active when exposed to photoactivation, namely when exposed to light. Photoactivatable groups typically comprise a protected reactive group, which upon exposure to light are de-protected, so as to generate a reactive group.

As used herein, the phrase "reactive group" describes a chemical moiety that is capable of interacting with another moiety. This interaction typically results in a bond formation between these moieties, whereby the bond can be, for example a covalent bond, a hydrogen bond, a coordinative bond, or a ionic bond.

Representative examples of reactive groups include, without limitation, amine, hydroxy, thiohydroxy, halo, alkoxy, thioalkoxy, aryloxy, thioaryloxy, carboxylate, phosphate, phosphonate, sulfate and sulfonate, as these terms are defined herein.

Depending on the intended use of the compound, the photoactivatable group is selected so as to generate a desired reactive group

Thus, for example, a photoactivatable group that comprises a carbamate can generate upon exposure to light amine as the reactive group.

The photoactivatable groups according to the present invention are preferably derived from photoactivatable compounds and therefore preferably include a residue of, for example, photoactivatable compounds that has light-absorbing characteristics such as 6-nitrovertaryl chloroformate, 6-nitrovertaryl carbonyl, 2-nitrotoluene, 2-nitroaniline, phenacyl, phenoxy, azidoaryl, sulfonic ester, desyl, p-hydroxyphenacyl, 7-methoxy coumarin, o-ethylacetophenone, 3,5-dimethylphenacyl, dimethyl dimethoxybenzyloxy carbonyl, 5-bromo-7-nitroindolinyl, o-hydroxy-α-methyl cinnamoyl and 2-oxymethylene anthraquinone.

When exposed to light such as, for example, UV, IR, or visible light or a monochromatic light of a predetermined wavelength, reactive groups, which are capable of binding a desired moiety, preferably a screenable moiety, as is detailed hereinunder, are generated.

As is demonstrated in the Examples section that follows, while reducing the present invention to practice, exemplary compounds according to the present invention have been readily prepared using a simple two-steps synthesis.

Hence, according to another aspect of the present invention there is provided a process of preparing the compounds described hereinabove. The process is effected by providing a polyethylene glycol (PEG) polymer having a molecular weight that ranges from 2000 grams/mol to 5000 grams/mol and containing a first reactive group and a second reactive group, such that the first reactive group is capable of reacting with a first compound which contains one or more functionalized groups as described hereinabove, and the second reactive group is capable of reacting with a second compound to thereby form the photoactivatable group; reacting the polymer with the first compound; and reacting the polymer with the second compound.

Thus, as a starting material, a bifunctional polyethylene glycol (PEG) polymer having a first and a second reactive groups, as this phrase is defined hereinabove, is selected. The first reactive group is selected so as to react and preferably form a covalent bond with a first compound, whereby the first compound is a compound from which the functionalized group described above is derived. The second reactive group is selected so as to react and preferably form a covalent bond with a second compound, whereby the second compound is a compound from which the photoactivatable group described above is derived.

Such bifunctional PEG polymers can be commercially available polymers or can be readily prepared using methods known to those skilled in the art.

Upon providing the starting material, it is reacted with the first compound, described hereinabove. Preferably the first compound comprises the functionatized group described above and an additional, third reactive group, which is selected so as to react with the first reactive group of the PEG polymer.

By reacting the first and the third reactive groups, a linking moiety as described hereinabove, linking between the functionalized group and the PEG polymer, is typically formed.

Representative examples of a first and third reactive groups suitable for use in this context of the present invention include, without limitation, hydroxy, thiohydroxy, alkoxy, thioalkoxy, aryloxy, thioaryloxy, halo, amine, cyano, nitro, azide, carboxylate, carbonyl, sulfonate, sulfate, phosphonate, phosphate, hydrazine, alkenyl and dienophile, as these terms are defined herein.

For example, amine and carboxylate can be selected as a first and a thirds reactive, so as to form an amide linking moiety. Hydroxy, alkoxy, aryloxy, thiohydroxy, thioalkoxy or thioarylosy and carboxylate can be selected as a first and a third reactive, so as to form an ester or a thioester linking moiety. Amine and sulfonate can be selected as a first and a third reactive, so as to form a sulfoamide linking moiety. Amine and halo can be selected as a first and a third reactive, so as to form an amine linking moiety. Alkenyls can participate in cycloaddition reactions or Michael additions, dienophils can participate in diels-adler reactions, and so forth.

In a representative example, the first reactive compound is carboxylate and the second reactive compound is amine, such that upon reacting a polymer having a free carboxylate reactive group with a first compound having a free amine group, an amide linking moiety is formed. Exemplary referred first compound according to such as embodiment of the present invention is an aminoalkyl trialkoxysilane such as, for example, aminopropyl triethoxysilane.

Upon being reacted with the first compound, the polymer is reacted with the second compound. The second compound is selected so as to form the photoactivatable group described above and preferably includes a photoactivatable moiety and a forth reactive group, whereby the second and the forth reactive groups are selected so as to form a photoactivatable group that generates the desired reactive group as detailed hereinabove and below.

In a preferred embodiment, the second reactive group is selected as being the reactive group, which is generated upon exposure to light. According to this embodiment, the second compound serves as a protecting group, which is removed upon exposure to light.

Representative examples of the second reactive group and the forth reactive group include, without limitation, hydroxy, thiohydroxy, alkoxy, thioalkoxy, aryloxy, thioaryloxy, halo, amine, cyano, nitro, azide, carboxylate, carbonyl, sulfonate, sulfate, phosphonate, phosphate, hydrazine, alkenyl and dienophile, as detailed hereinabove.

Representrative examples of second compounds are listed hereinabove.

The process described above can alternatively be effected by first reacting the polymer with the second compound and thereafter with the first compound.

As discussed hereinabove, the compounds of the present invention are particularly useful for attaching various moieties to a solid substrate, and, preferably, to a pre-selected area of a solid substrate.

Hence, according to an additional aspect of the present invention there is provided a process of attaching a screenable moiety to at least one pre-selected area of a solid substrate. The process, according to this aspect of the present invention, is effected by:
providing a solid substrate having at least one functional group on a surface thereof;
providing a compound having a functionalized group capable of binding to said solid substrate, said functionalized group comprising at least one reactive silyl group, a photoactivatable group capable of generating a reactive group upon exposure to a light source, said reactive group being capable of binding said screenable moiety, and a polyethylene glycol (PEG) polymer capable of forming a monolayer on said solid substrate and having said functionalized group and said photoactivatable group attached thereto, said PEG polymer having a molecular weight that ranges from 2000 grams/mol to 5000 grams/mol;
contacting said compound with said solid substrate, in a one-step reaction, by reacting said functionalized group with said functional group on said surface of the solid substrate, to thereby provide a solid substrate having a plurality of monolayers of said polymer attached thereto, wherein each of said monolayers terminates with said photoactivatable group;
exposing said at least one pre-selected area of said solid substrate having said monolayer of said polymer attached thereto to a light, to thereby generate said reactive group from said photoactivatable group at said pre-selected area; and
binding said screenable moiety to said reactive group, thereby attaching the screenable moiety to the pre-selected area of the solid support.

Solid substrates useful in practicing the present invention can be made of any stable material, or combination of materials. Moreover, useful substrates can be configured to have any convenient geometry or combination of structural features. The substrates can be either rigid or flexible and can be either optically transparent or optically opaque. The substrates can also be electrical insulators, conductors or semiconductors. Further the substrates can be substantially impermeable to liquids, vapors and/or gases or, alternatively, the substrates can be substantially permeable to one or more of these classes of materials.

Essentially, any conceivable substrate may be employed in the invention. The substrate may be biological, nonbiological, organic, inorganic, or a combination of any of these, existing as particles, strands, precipitates, gels, sheets, tubing, spheres, containers, capillaries, pads, slices, films, plates, slides, etc. The substrate may have any convenient shape, such as a disc, square, sphere, circle, etc. The substrate is preferably flat but may take on a variety of alternative surface configurations. For example, the substrate may contain raised or depressed regions on which the synthesis takes place. The substrate and its surface preferably form a rigid support on which to carry out the reactions described herein. The substrate and its surface are also chosen to provide appropriate light-absorbing characteristics.

The materials forming the substrate are utilized in a variety of physical forms such as films, supported powders, glasses, crystals and the like. For example, a substrate can consist of a single inorganic oxide or a composite of more than one inorganic oxide. When more than one component is used to form a substrate, the components can be assembled in, for example a layered structure (i.e., a second oxide deposited on a first oxide) or two or more components can be arranged in a contiguous non-layered structure. Moreover, one or more components can be admixed as particles of various sizes and deposited on a support, such as a glass, quartz or metal sheet. Further, a layer of one or more components can be intercalated between two other substrate layers (e.g., metal-oxide-metal, metal-oxide-crystal). Those of skill in the art are able to select an appropriately configured substrate, manufactured from an appropriate material for a particular application.

Exemplary substrate materials include, but are not limited to, inorganic crystals, inorganic glasses, inorganic oxides, metals, organic polymers and combinations thereof. Inorganic glasses and crystals of use in the substrate include, but are not limited to, LiF, NaF, NaCl, KBr, KI, CaF₂, MgF₂, HgF₂, BN, AsS₃, ZnS, Si₃N₄ and the like. The crystals and glasses can be prepared by art standard techniques. See, for example, Goodman, Crystal Growth Theory and Techniques, Plenum Press, New York 1974. Alternatively, the crystals can be purchased commercially (e.g., Fischer Scientific). Inorganic oxides of use in the present invention include, but are not limited to, Cs₂O, Mg(OH)₂, TiO₂, ZrO₂, CeO₂, Y₂O₃, Cr₂O₃, Fe₂O₃, NiO, ZnO, Al₂O₃, SiO₂ (glass), quartz, In₂O₃, SnO₂, PbO₂ and the like. Metals of use in the substrates of the invention include, but are not limited to, gold, silver, platinum, palladium, nickel, copper and alloys and composites of these metals.

The metal can be used as a crystal, a sheet or a powder. In those embodiments in which the metal is layered with another substrate component, the metal can be deposited onto the other substrate by any method known to those of skill in the art including, but not limited to, evaporative deposition, sputtering and electroless deposition.

Organic polymers that form useful substrates include, for example, polyalkenes (e.g., polyethylene, polyisobutene, polybutadiene), polyacrylics (e.g., polyacrylate, polymethyl methacrylate, polycyanoacrylate), polyvinyls (e.g., polyvinyl alcohol, polyvinyl acetate, polyvinyl butyral, polyvinyl chloride), polystyrenes, polycarbonates, polyesters, polyurethanes, polyamides, polyimides, polysulfone, polysiloxanes, polyheterocycles, cellulose derivative (e.g., methyl cellulose, cellulose acetate, nitrocellulose), polysilanes, fluorinated polymers, epoxies, polyethers and phenolic resins.

In a preferred embodiment, the substrate material is substantially non-reactive with the screenable moiety, thus preventing non-specific binding between the substrate and the moiety or other components of an assay mixture. Methods of coating substrates with materials to prevent non-specific binding are generally known in the art. Exemplary coating agents include, but are not limited to cellulose, bovine serum albumin, and poly(ethyleneglycol). The proper coating agent for a particular application will be apparent to one of skill in the art.

In a further preferred embodiment, the substrate material is substantially non-fluorescent or emits light of a wavelength range that does not interfere with the photoactivation. Exemplary low-background substrates include those disclosed by Cassin et al., U.S. Patents No. 5,910,287 and Pham et al., U.S. Patent No. 6,063,338.

As discussed hereinabove, the solid substrate and the compound of the present invention are selected such that upon contacting the polymer with the substrate, a self-assembled monolayered film of the polymer forms on the substrate surface, in a one-step reaction. Such a process is therefore highly advantageous as compared with the presently known multi-step technique since it provides for a simple, cost-effective technique, which further provides for improved signal-to-noise ratio.

The contacting procedure is preferably effected by incubating the compound of the present invention with the selected solid substrate, preferably in the presence of an organic solvent such as, for example, toluene.

Once a monolayered film of the polymer is deposited on the substrate surface, the reactive group for binding a screenable moiety can be generated by exposing a pre-selected area of the substrate to light.

Depending on the selected photoactivatable group and the active wavelength in which it is active, the light can be a UV, IR or visible light, or, optionally and preferably, the light can be a monochromatic light of a predetermined wavelength.

Exposure a pre-selected area of the substrate to light is preferably effected using a photo mask to illuminate selected regions the substrate. However, other techniques may also be used. For example, the substrate may be translated under a modulated laser or diode light source. Such techniques are discussed in, for example, U.S. Pat. No. 4,719,615 (Feyrer et al.). In alternative embodiments a laser galvanometric scanner is utilized. In other embodiments, the synthesis may take place on or in contact with a conventional liquid crystal (referred to herein as a "light valve") or fiber optic light sources. By appropriately modulating liquid crystals, light may be selectively controlled so as to permit light to contact selected regions of the substrate. Alternatively, synthesis may take place on the end of a series of optical fibers to which light is selectively applied. Other means of controlling the location of light exposure will be apparent to those of skill in the art.

The substrate may be irradiated either in contact or not in contact with a solution and is, preferably, irradiated in contact with a solution. The solution may contain reagents to prevent the by-products formed by irradiation. Such by-products might include, for example, carbon dioxide, nitrosocarbonyl compounds, styrene derivatives, indole derivatives, and products of their photochemical reactions. Alternatively, the solution may contain reagents used to match the index of refraction of the substrate. Reagents added to the solution may further include, for example, acidic or basic buffers, thiols, substituted hydrazines and hydroxylamines, or reducing agents (e.g., NADH).

In an exemplary embodiment, exposing the substrate to light is effected so as to provide a patterned substrate in which reactive groups are generated according to a pre-selected pattern. The pattern can be printed directly onto the substrate or, alternatively, a "lift off' technique can be utilized. In the lift off technique, a patterned resist is laid onto the substrate or onto the light source. Resists are known to those of skill in the art. See, for example, Kleinfield et al., J. Neurosci. 8:4098-120 (1998). In some embodiments, following removal of the resist, a second pattern is printed onto the substrate on those areas initially covered by the resist; a process that can be repeated any selected number of times with different components to produce an array having a desired format.

Once the reactive group is generated at a pre-selected area or pattern, and preferably concomitant therewith, it is reacted so as to bind a desired screenable moiety.

Binding the screenable moiety can be effected by directly attaching the moiety to the reactive group.

Alternatively, binding the screenable moiety is effected via a mediating moiety. As used herein, the phrase "mediating moiety" describes a mediating agent or a plurality of mediating agents being linked therebetween that may bind to both the reactive group and the screenable moiety and thus mediate the binding of the screenable moiety to the reactive group.

The mediating moiety can thus be a bifunctional moiety, having two reactive groups, each independently capable of reacting with the reactive group attached to the substrate or the screenable moiety. Alternatively, the mediating moiety can comprise two or more moieties, whereby the first moiety can be attached to the reactive group and to a second mediating moiety, whereby the second mediating moiety can bind the screenable moiety.

Optionally and preferably, the mediating moiety comprises an affinity pair, such as, for example, the biotin-avidin affinity pair. The biotin-avidin affinity pair is highly useful for integrating biomolecules on the substrate.

Alternatively, the mediating moiety can comprise biotin. When attached to the reactive group, biotin can bind a variety of chemical and biological substances that are capable of reacting with the free carboxylic group thereof.

Using the technology described above, arrays, and preferably microarrays, of screenable moieties can be efficiently prepared.

Thus, according to a further aspect of the present invention there is provided a process of preparing an array of screenable moieties. The process is effected by providing a solid substrate having a plurality of functional groups on a surface thereof; providing a compound having a functionalized group capable of binding to said solid substrate, said functionalized group comprising at least one reactive silyl group, a photoactivatable group capable of generating a reactive group upon exposure to a light source, said reactive group being capable of binding a screenable moiety; and a polyethylene glycol (PEG) polymer capable of forming a monolayer on said solid substrate and having said functionalized group and said photoactivatable group attached thereto, said PEG polymer has a molecular weight that ranges from 2000 grams/mol to 5000 grams/mol; contacting said compound to said solid substrate by reacting said functionalized group with said functional groups on said surface, to thereby provide, in a one-step reaction, a solid substrate having a plurality of monolayers of said polymer attached thereto, wherein each of said monolayers terminates with said photoactivatable group; exposing a first pre-selected area of said solid substrate having said monolayer of said polymer attached thereto to a light, to thereby generate a reactive group from said photoactivatable group at said first pre-selected area; and binding a screenable moiety to said reactive group at said first pre-selected area; exposing a second pre-selected area of said solid substrate having said monolayer of said polymer attached thereto to a light, to thereby generate a reactive group from said photoactivatable group at said second pre-selected area; binding a second screenable moiety to said reactive group at said second pre-selected area; consecutively exposing an Nth pre-selected area of said solid substrate having said monolayer of said polymer attached thereto to a light, to thereby generate a reactive group at said Nth pre-selected area; and binding an Nth screenable moiety to said reactive group at said Nth pre-selected area, thereby providing an array of screenable moieties.

Exposing to light of the different areas of the substrate can be effected by exchanging the photo mask in each exposure and/or by displacing the patterning area. The number of cycles N typically ranges from 2 to 2000, depending on the intended application of the array.

Exemplary pre-selected areas of the solid substrate exposed to light typically range between 1 x 10⁻² cm² to 1x10⁻² cm² and 1x 10⁻⁵ cm² to 1 x 10⁻⁵ cm².

The arrays of screenable moieties produced by the technology set forth herein therefore comprise a solid substrate having a plurality of monolayers of a polyethylene glycol (PEG) polymer applied thereon, said PEG polymer has a molecular weight that ranges from 2000 grams/mol to 5000 grams/mol and having a functionalized group and a photoactivatable group attached thereto, said functionalized group being capable of binding to said solid substrate, and comprising at least one reactive silyl group, said photoactivatable group being capable of generating a reactive group upon exposure to a light source, said reactive group being capable of binding said screenable moiety, wherein each of said plurality of monolayers of said PEG polymer is applied on said solid substrate, in a one-step reaction, by reacting a functionalized group attached to said polymer with a functional group on a surface of said solid substrate, said functionalized group comprising at least one reactive silyl group; and a plurality of screenable moieties being bound to said plurality of monolayers at pre-selected areas of said solid substrate, such that each of said screenable moieties is bound to each of said monolayers at a pre-selected area of said solid substrate upon exposure to light of a plurality of monolayers of said PEG polymer in which each monolayer terminates with a photoactivatable group, at said pre-selected area of said solid substrate, wherein said exposure to light generates a reactive group which binds said screenable moiety.

The present invention relies in part on the ability to synthesize or attach specific screenable moieties at known locations on a substrate, typically a single substrate. In particular, the present invention provides the ability to prepare a substrate having a very high density matrix pattern of positionally defined specific screenable moieties. The moieties are capable of interacting with corresponding targets while attached to the substrate, e.g., solid phase interactions, and by appropriate labeling of these targets, the sites of the interactions between the target and the specific moieties may be derived. Because the screenable moieties are positionally defined, the sites of the interactions will define the specificity of each interaction. As a result, a map of the patterns of interactions with specific moieties on the substrate is convertible into information on the specific interactions taking place, e.g., the recognized features. Where the screenable moieties recognize a large number of possible features, this system allows the determination of the combination of specific interactions which exist on the target molecule. Where the number of features is sufficiently large, the identical same combination, or pattern, of features is sufficiently unlikely that a particular target molecule may often be uniquely defined by its features.

The screenable moieties in the arrays according to the present invention can be, depending on the intended use of the array, a plurality of one screenable moiety, and, optionally and preferably, distinguished screenable moieties.

As used herein, the phrase "screenable moiety" describes a moiety that is characterized by diversity and activity which allows its screening, due to its potential interaction with a target molecule.

Screenable moieties according to the present invention can therefore be biological moieties or chemical moieties. Such a biological or chemical moiety can be, for example, a drug or a drug target. A chemical moiety that functions as a drug for example, can be a small organic molecule, which can, for example, inhibit or activate a process. A biological moiety that functions as a drug can be, for example, a growth factor or hormone.

Representative examples of biological moieties that are suitable for use in the context of the present invention include, without limitation, peptides, proteins, glycoproteins, proteoglycans, nucleic acids, polynucleotides, oligonucleotides, antibodies, carbohydrates, lipids, cells, microorganisms, enzymes, hormones, steroids, second messengers and growth factors.

The term "peptide" as used herein encompasses native peptides (either degradation products, synthetically synthesized peptides or recombinant peptides) and peptidomimetics (typically, synthetically synthesized peptides), as well as as peptoids and semipeptoids which are peptide analogs, which may have, for example, modifications rendering the peptides more stable while in a body or more capable of penetrating into cells. Such modifications include, but are not limited to N terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992).

Peptide bonds (-CO-NH-) within the peptide may be substituted, for example, by N-methylated bonds (-N(CH3)-CO-), ester bonds (-C(R)H-C-O-O-C(R)-N-), ketomethylen bonds (-CO-CH2-), α-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), peptide derivatives (-N(R)-CH2-CO-), wherein R is the "normal" side chain, naturally presented on the carbon atom.

The term "oligonucleotide" refers to a single stranded or double stranded oligomer or polymer of ribonucleic acid (RNA) or deoxyribonucleic acid (DNA) or mimetics thereof. This term includes oligonucleotides composed of naturally-occurring bases, sugars and covalent internucleoside linkages (e.g., backbone) as well as oligonucleotides having non-naturally-occurring portions which function similarly.

Oligonucleotides designed according to the teachings of the present invention can be generated according to any oligonucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art.

Oligonucleotides used according to this aspect of the present invention are those having a length selected from a range of 10 to about 200 bases preferably 15-150 bases, more preferably 20-100 bases, most preferably 20-50 bases.

The oligonucleotides of the present invention may comprise heterocylic nucleosides consisting of purines and the pyrimidines bases, bonded in a 3' to 5' phosphodiester linkage. Oligonucleotides can be modified in either backbone, internucleoside linkages or bases. Such modifications can oftentimes facilitate oligonucleotide uptake and resistivity to intracellular conditions.

Alternatively, modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. Other oligonucleotides which can be used according to the present invention, are those modified in both sugar and the internucleoside linkage, i.e., the backbone of the nucleotide units are replaced with novel groups. The base units are maintained for complementation with the appropriate polynucleotide target. Other backbone modifications, which can be used in the present invention are disclosed in U.S. Pat. No: 6,303,374. Oligonucleotides of the present invention may also include base modifications or substitutions as disclosed in U.S. Pat. No: 3,687,808, The Concise Encyclopedia Of Polymer Science And Engineering, pages 858-859, Kroschwitz, J. I., ed. John Wiley & Sons, 1990, Englisch et al., Angewandte Chemie, International Edition, 1991, 30, 613, Sanghvi, Y. S., Chapter 15, Antisense Research and Applications, pages 289-302, and Crooke, S. T. and Lebleu, B. , ed., CRC Press, 1993.

Another modification of the oligonucleotides of the invention involves chemically linking to the oligonucleotide one or more moieties or conjugates, which enhance the activity, cellular distribution or cellular uptake of the oligonucleotide. Such moieties include but are not limited to lipid moieties such as a cholesterol moiety, cholic acid, a thioether, e.g., hexyl-S-tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxycholesterol moiety, as disclosed in U.S. Pat. No: 6,303,374.

It is not necessary for all positions in a given oligonucleotide molecule to be uniformly modified, and in fact more than one of the aforementioned modifications may be incorporated in a single compound or even at a single nucleoside within an oligonucleotide.

Oligonucleotides also includes antisense molecules, which are chimeric molecules. "Chimeric antisense molecules" are oligonucleotides, which contain two or more chemically distinct regions, each made up of at least one nucleotide. These oligonucleotides typically contain at least one region wherein the oligonucleotide is modified so as to confer upon the oligonucleotide increased resistance to nuclease degradation, increased cellular uptake, and/or increased binding affinity for the target polynucleotide. An additional region of the oligonucleotide may serve as a substrate for enzymes capable of cleaving RNA:DNA or RNA:RNA hybrids. Chimeric antisense molecules may be formed as composite structures of two or more oligonucleotides or modified oligonucleotides. Chimeric oligonucleotides can also comprise a ribozyme sequence.

Alternatively, oligonucleotides of the present invention may include small interfering duplex oligonucleotides [i.e., small interfering RNA (siRNA)], which direct sequence specific degradation of mRNA through the previously described mechanism of RNA interference (RNAi) [Hutvagner and Zamore (2002) Curr. Opin. Genetics and Development 12:225-232].

As used herein, the phrase "duplex oligonucleotide" refers to an oligonucleotide structure or mimetics thereof, which is formed by either a single self-complementary nucleic acid strand or by at least two complementary nucleic acid strands. The "duplex oligonucleotide" of the present invention can be composed of double-stranded RNA (dsRNA), a DNA-RNA hybrid, single-stranded RNA (ssRNA), isolated RNA (i.e., partially purified RNA, essentially pure RNA), synthetic RNA and recombinantly produced RNA.

Preferably, the specific small interfering duplex oligonucleotide of the present invention is an oligoribonucleotide composed mainly of ribonucleic acids. Instructions for generation of duplex oligonucleotides capable of mediating RNA interference are provided in www.ambion.com.

The term "antibody" as used herein includes intact molecules as well as functional fragments thereof, such as Fab, F(ab')₂, and Fv that are capable of binding to macrophages. These functional antibody fragments are defined as follows:
(1) Fab, the fragment which contains a monovalent antigen-binding fragment of an antibody molecule, can be produced by digestion of whole antibody with the enzyme papain to yield an intact light chain and a portion of one heavy chain;
(2) Fab', the fragment of an antibody molecule that can be obtained by treating whole antibody with pepsin, followed by reduction, to yield an intact light chain and a portion of the heavy chain; two Fab' fragments are obtained per antibody molecule;
(3) (Fab')₂, the fragment of the antibody that can be obtained by treating whole antibody with the enzyme pepsin without subsequent reduction; F(ab')₂ is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule. Methods of making these fragments are known in the art. See for example, Harlow and Lane, Antibodies: A Laboratory Manual, Cold Spring Harbor Laboratory, New York, 1988.

Growth factors are hormones which have numerous functions, including regulation of adhesion molecule production, altering cellular proliferation, increasing vascularization, enhancing collagen synthesis, regulating bone metabolism and altering migration of cells into given area. Non-limiting examples of growth factors include insulin-like growth factor-1 (IGF-1), transforming growth factor-β (TGF-β), a bone morphogenic protein (BMP) and the like.

Suitable hormones for use in the context of the present invention include, for example, androgenic compounds and progestin compounds. Representative examples of androgenic compounds include, without limitation, methyltestosterone, androsterone, androsterone acetate, androsterone propionate, androsterone benzoate, androsteronediol, androsteronediol-3-acetate, androsteronediol-17-acetate, androsteronediol 3-17-diacetate, androsteronediol-17-benzoate, androsteronedione, androstenedione, androstenediol, dehydroepiandrosterone, sodium dehydroepiandrosterone sulfate, dromostanolone, dromostanolone propionate, ethylestrenol, fluoxymesterone, nandrolone phenpropionate, nandrolone decanoate, nandrolone furylpropionate, nandrolone cyclohexane-propionate, nandrolone benzoate, nandrolone cyclohexanecarboxylate, androsteronediol-3-acetate-1-7-benzoate, oxandrolone, oxymetholone, stanozolol, testosterone, testosterone decanoate, 4-dihydrotestosterone, 5α-dihydrotestosterone, testolactone, 17α-methyl-19-nortestosterone and pharmaceutically acceptable esters and salts thereof, and combinations of any of the foregoing.

Representative examples of progestin compounds include, without limitation, desogestrel, dydrogesterone, ethynodiol diacetate, medroxyprogesterone, levonorgestrel, medroxyprogesterone acetate, hydroxyprogesterone caproate, norethindrone, norethindrone acetate, norethynodrel, allylestrenol, 19-nortestosterone, lynoestrenol, quingestanol acetate, medrogestone, norgestrienone, dimethisterone, ethisterone, cyproterone acetate, chlormadinone acetate, megestrol acetate, norgestimate, norgestrel, desogrestrel, trimegestone, gestodene, nomegestrol acetate, progesterone, 5α-pregnan-3β,20α-diol sulfate, 5α-pregnan-3β,20β-diol sulfate, 5α-pregnan-3β-ol-20-one, 16,5α-pregnen-3β-ol-20-one, 4-pregnen-20β-ol-3-one-20-sulfate, acetoxypregnenolone, anagestone acetate, cyproterone, dihydrogesterone, flurogestone acetate, gestadene, hydroxyprogesterone acetate, hydroxymethylprogesterone, hydroxymethyl progesterone acetate, 3-ketodesogestrel, megestrol, melengestrol acetate, norethisterone and mixtures thereof.

Representative examples of chemical moieties that are suitable for used in the context of the present invention include, without limitation, chelators, ligands, substrates and inhibitors.

As used herein, the term "chelator" describes a moiety that is capable of chelating another moiety. Exemplary chelators include, without limitation, polyamines.

As used herein, the term "ligand" describes a moiety that is capable of forming coordinative interaction with another moiety. Exemplary ligands include, without limitation, porphyrins, derivatives and analogs thereof.

The term "substrate" as used in this context of the present invention describes any substance with which another moiety can interact. These may include, for example, substrates that interact with enzymes, or with a chemical reagent.

The term "inhibitor" describes a chemical moiety that is capable of inhibiting an activity of a biological moiety (e.g., enzymes).

Each of these moieties can be detected as a vapor or a liquid. The moiety can be present as a component in a mixture of structurally unrelated compounds, an assay mixture, racemic mixtures of stereoisomers, non-racemic mixtures of stereoisomers, mixtures of diastereomers, mixtures of positional isomers or as a pure compound.

The screenable moiety to be attached on the array according to the present invention is selected in accordance with the intended use of the array. Thus, for example, for synthesizing peptides or proteins, the selected screenable moiety comprises genes.

Polymers such as nucleic acids or polypeptides can be synthesized in situ using, for example, photolithography and other masking techniques whereby molecules are synthesized in a step-wise manner with incorporation of monomers at particular positions being controlled by means of masking techniques and photolabile reactants. For example, U.S. Pat. No. 5,837,832 describes a method for producing DNA arrays immobilised to silicon substrates based on very large scale integration technology. In particular, U.S. Pat. No. 5,837,832 describes a strategy called "tiling" to synthesize specific sets of probes at spatially-defined locations on a substrate. Light directed synthesis can also be carried out by using a Digital Light Micromirror chip (Texas Instruments) as described (Singh-Gasson et al., (1999) Nature Biotechnology 17:974-978). Instead of using photo-deprotecting groups which are directly processed by light, conventional deptotecting groups such as dimethoxy trityl can be employed with light directed methods where for example a photoacid is generated in a spatially addressable way which selectively deprotects the DNA monomers (McGall et al PNAS 1996 93: 1355-13560; Gao et al J. Am. Chem Soc. 1998 120: 12698-12699).

An alternative approach for in situ synthesis of biomolecular arrays (DNA, RNA, protein) is described in Ramachandran et al. 2004 Science 305:86. Basically, by in vitro translation, transcription or reverse transcription of the screenable moiety from an immobilized source, followed by affinity based immobilization of the synthesized product. For example, a protein array is generated by immobilizing DNA onto the support and then protein targets are translated in the presence of a cell-free translational system (see Example 4 of the Examples section which follows). To avoid diffusion (migration) of the translated product, an epitope tag is encoded in frame to the target protein by the DNA molecule. This tag allows the proteins to be immobilized in-situ (by co-immobilizing the cognate antibody with the DNA molecule). This approach obviates the need to purify proteins, avoids protein stability problems during storage and captures sufficient protein for functional studies.

A screenable moiety can also be derived from any sort of synthetic or biological source, including body fluids such as blood, serum, saliva, urine, seminal fluid, seminal plasma, lymph, and the like. It also includes extracts from biological samples, such as cell lysates, cell culture media, or the like.

The screenable moiety can be labeled with a fluorophore or other detectable group either directly (e.g., Cy3 labeled DNA, see Example 4 of the Examples section which follows) or indirectly through interacting with a second species to which a detectable group is bound. When a second labeled species is used as an indirect labeling agent, it is selected from any species that is known to interact with the target species. Preferred second labeled species include, but are not limited to, antibodies, aptazymes, aptamers, streptavidin, and biotin.

The screenable moiety can be labeled either before or after it interacts with the reactive group. The screenable moiety can be labeled with a detectable group or more than one detectable group.

In particular, the methodology is applicable to sequencing polynucleotides. The specific sequence recognition reagents will typically be oligonucleotide probes which hybridize with specificity to subsequences found on the target sequence. A sufficiently large number of those probes allows the fingerprinting of a target polynucleotide or the relative mapping of a collection of target polynucleotides, as described in greater detail below.

In the high resolution fingerprinting provided by a saturating collection of probes which include all possible subsequences of a given size, e.g., 10-mers, collating of all the subsequences and determination of specific overlaps will be derived and the entire sequence can usually be reconstructed.

Although a polynucleotide sequence analysis is a preferred embodiment, for which the specific reagents are most easily accessible, the invention is also applicable to analysis of other polymers, including polypeptides, carbohydrates, and synthetic polymers, including alpha-, .beta-, and omega-amino acids, polyurethanes, polyesters, polycarbonates, polyureas, polyamides, polyethyleneimines, polyarylene sulfides, polysiloxanes, polyimides, polyacetates, and mixed polymers. Various optical isomers, e.g., various D- and L-forms of the monomers, may be used.

Using the teachings of the present invention, screenable moieties are bound to the solid substrate at a submicron (< µm) resolution.

The platform technology reported here is a step toward simplifying bio-integration, requiring no more than a commonly available fluorescent microscope.

The array of the present invention is useful in performing assays of substantially any format including, but not limited to chromatographic capture, including retentate chromatography, immunoassays, competitive assays, DNA or RNA binding assays, fluorescence in situ hybridization (FISH), protein and nucleic acid profiling assays, sandwich assays and the like. Those of skill in the art will appreciate that the method of the invention is broadly applicable to any assay technique for detecting the presence and/or amount of a target.

The array of the invention is useful in applications such as sequential extraction of moieties from a solution, progressive resolution of moieties in a sample, preparative purification of a moiety, making probes for specific detection of moieties, methods for identifying proteins, methods for assembling multimeric molecules, methods for performing enzyme assays, methods for identifying moieties that are differentially expressed between biological sources, methods for identifying ligands for a receptor, methods for drug discovery (e.g., screening assays), and methods for generating agents that specifically bind a moiety.

In other applications, array-based assays based on specific binding reactions are useful to detect a wide variety of screenable moieties such as drugs, hormones, enzymes, proteins, antibodies, and infectious agents in various biological fluids and tissue samples. In general, the assays consist of a screenable moiety, its attachment to the solid substrate as described herein, and a means of detecting the target after its immobilization by the binding functionality (e.g., a detectable label). Immunological assays involve reactions between immunoglobulins (antibodies), which are capable of binding with specific antigenic determinants of various compounds and materials (antigens). Other types of reactions include binding between avidin and biotin, protein A and immunoglobulins, lectins and sugar moieties and the like. See, for example, U.S. Pat. No. 4,313,734 , issued to Leuvering; U.S. Pat. No. 4,435,504, issued to Zuk; U.S. Pat. Nos. 4,452,901 and 4,960,691, issued to Gordon; and U.S. Pat. No. 3,893,808, issued to Campbell.

The present invention provides an array useful for performing assays that confirm the presence or absence of a moiety in a sample and for quantitating a moiety in a sample. An exemplary assay format with which the invention can be used is an immunoassay, for example, competitive assays, and sandwich assays. The invention is further illustrated using these two assay formats. The focus of the following discussion on competitive assays and sandwich assays is for clarity of illustration and is not intended to either define or limit the scope of the invention. Those of skill in the art will appreciate that the invention described herein can be practiced in conjunction with a number of other assay formats.

In an exemplary competitive binding assay, two species, one of which is the moiety of interest, compete for attachment to the substrate. After an incubation period, unbound materials are washed off and the amount of moiety or other species bound to the substrate is compared to reference amounts for determination of the moiety, or other species concentration in the assay mixture. Other competitive assay motifs using labeled target and/or labeled binding functionality and/or labeled reagents will be apparent to those of skill in the art.

In addition to detecting an interaction between a binding functionality and a target, it is frequently desired to quantitate the magnitude of the affinity between two or more binding partners. The format of an assay for extracting affinity data for two molecules can be understood by reference to an embodiment in which a ligand that is known to bind to a receptor is displaced by an antagonist to that receptor. Other variations on this format will be apparent to those of skill in the art. The competitive format is well known to those of skill in the art. See, for example, U.S. Pat. Nos. 3,654,090 and 3,850,752.

The binding of an antagonist to a receptor can be assayed by a competitive binding method using a ligand for that receptor and the antagonist. One of the three binding partners (i.e., the ligand, antagonist or receptor) is attached to the substrate. In an exemplary embodiment, the receptor is attached. Various concentrations of ligand are added to different array regions. A detectable antagonist is then applied to each region to a chosen final concentration. The treated array will generally be incubated at room temperature for a preselected time. The receptor-bound antagonist can be separated from the unbound antagonist by filtration, washing or a combination of these techniques. Bound antagonist remaining on the array can be measured as discussed herein. A number of variations on this general experimental procedure will be apparent to those of skill in the art.

Competition binding data can be analyzed by a number of techniques, including nonlinear least-squares curve fitting procedure. When the ligand is an antagonist for the receptor, this method provides the IC50 of the antagonist (concentration of the antagonist which inhibits specific binding of the ligand by 50% at equilibrium). The IC50 is related to the equilibrium dissociation constant (Ki) of the antagonist based on the Cheng and Prusoff equation: Ki=IC50/(1+L/Kd), where L is the concentration of the ligand used in the competitive binding assay, and Kd is the dissociation constant of the ligand as determined by Scatchard analysis. These assays are described, among other places, in Maddox et al., J Exp Med., 158: 1211 (1983); Hampton et al., Serological Methods, A Laboratory Manual, APS Press, St. Paul, Minn., 1990.

The array of the present invention can also be used to screen libraries of compounds, such as combinatorial libraries. The synthesis and screening of chemical libraries to identify compounds, which have novel bioactivities, and material science properties is now a common practice. Libraries that have been synthesized include, for example, collections of oligonucleotides, oligopeptides, and small and large molecular weight organic or inorganic molecules. See, Moran et al., PCT Publication WO 97/35198, published Sep. 25, 1997; Baindur et al., PCT Publication WO 96/40732, published Dec. 19, 1996; Gallop et al., J. Med. Chem. 37:1233-51 (1994).

Virtually any type of compound library can be probed using the array of the invention, including peptides, nucleic acids, saccharides, small and large molecular weight organic and inorganic compounds. The libraries synthesized comprise more than 10 unique compounds, preferably more than 100 unique compounds and more preferably more than 1000 unique compounds.

A binding domain of a receptor, for example, serves as the focal point for a drug discovery assay, where, for example, the receptor is immobilized, and incubated both with agents (i.e., ligands) known to interact with the binding domain thereof, and a quantity of a particular drug or inhibitory agent under test. The extent to which the drug binds with the receptor and thereby inhibits receptor-ligand complex formation can then be measured. Such possibilities for drug discovery assays are described herein . ther focal points and appropriate assay formats will be apparent to those of skill in the art.

The presence of the screenable moiety and attachment of a binding agent thereto can be detected by the use of microscopes, spectrometry, electrical techniques and the like. For example, light in the visible region of the spectrum is used to illuminate details of the surface of the substrate (e.g., reflectance, transmittance, birefringence, diffraction, etc.). Alternatively, the light can be passed through the substrate and the amount of light transmitted, absorbed or reflected can be measured. The device can utilize a backlighting device such as that described in U.S. Pat. No. 5,739,879. Light in the ultraviolet and infrared regions is also of use in the present invention.

For the detection of low concentrations of screenable moieties in the field of diagnostics, the methods of chemiluminescence and electrochemiluminescence are gaining wide-spread use. These methods of chemiluminescence and electrochemiluminescence provide a means to detect low concentrations of moieties by amplifying the number of luminescent molecules or photon generating events many-fold, the resulting "signal amplification" then allowing for detection of low concentration moieties.

A fluorescent label is useful to label one or more assay component or region of the array. Fluorescent labels have the advantage of requiring few precautions in handling, and being amenable to high-throughput visualization techniques (optical analysis including digitization of the image for analysis in an integrated system comprising a computer). Preferred labels are typically characterized by one or more of the following: high sensitivity, high stability, low background, low environmental sensitivity and high specificity in labeling. Many fluorescent labels are commercially available from the SIGMA chemical company (Saint Louis, Mo.), Molecular Probes (Eugene, Oreg.), R&D systems (Minneapolis, Minn.), Pharmacia LKB Biotechnology (Piscataway, N.J.), CLONTECH Laboratories, Inc. (Palo Alto, Calif.), Chem Genes Corp., Aldrich Chemical Company (Milwaukee, Wis.), Glen Research, Inc., GIBCO BRL Life Technologies, Inc. (Gaithersburg, Md.), Fluka Chemica-Biochemika Analytika (Fluka Chemie AG, Buchs, Switzerland), and Applied Biosystems (Foster City, Calif.), as well as many other commercial sources known to one of skill. Furthermore, those of skill in the art will recognize how to select an appropriate fluorophore for a particular application and, if it not readily available commercially, will be able to synthesize the necessary fluorophore de novo or synthetically modify commercially available fluorescent compounds to arrive at the desired fluorescent label.

In addition to small molecule fluorophores, naturally occurring fluorescent proteins and engineered analogues of such proteins are useful in the present invention. Such proteins include, for example, green fluorescent proteins of cnidarians (Ward et al., Photochem. Photobiol. 35:803-808 (1982); Levine et al., Comp. Biochem. Physiol., 72B:77-85 (1982)), yellow fluorescent protein from Vibrio fischeri strain (Baldwin et al., Biochemistry 29:5509-15 (1990)), Peridinin-chlorophyll from the dinoflagellate Symbiodinium sp. (Morris et al., Plant Molecular Biology 24:673:77 (1994)), phycobiliproteins from marine cyanobacteria, such as Synechococcus, e.g., phycoerythrin and phycocyanin (Wilbanks et al., J. Biol. Chem. 268:1226-35 (1993)), and the like.

Microscopic techniques of use include, but are not limited to, simple light microscopy, confocal microscopy, polarized light microscopy, atomic force microscopy (Hu et al., Langmuir 13:5114-5119 (1997)), scanning tunneling microscopy (Evoy et al., J. Vac. Sci. Technol A 15:1438-1441, Part 2 (1997)), and the like.

Spectroscopic techniques of use include, for example, infrared spectroscopy (Zhao et al., Langmuir 13:2359-2362 (1997)), raman spectroscopy (Zhu et al., Chem. Phys. Lett. 265:334-340 (1997)). X-ray photoelectron spectroscopy (Jiang et al., Bioelectroch. Bioener. 42:15-23 (1997)) and the like. Visible and ultraviolet spectroscopies are also of use.

Other useful techniques include, for example, surface plasmon resonance (Evans et al., J. Phys. Chem. B 101:2143-2148 (1997), ellipsometry (Harke et al., Thin Solid Films 285:412-416 (1996)), impedometric methods (Rickert et al., Biosens. Bioelectron. 11:757:768 (1996)), and the like.

In addition, the Polymerase Chain Reaction (PCR) and other related techniques have gained wide use for amplifying the number of nucleic acid moieties in a sample. By the addition of appropriate enzymes, reagents, and temperature cycling methods, the number of nucleic acid molecules can be amplified such that they can be detected by most known detection means.

Of particular interest is the use of mass spectrometric techniques to detect moieties attached to the substrate, particularly those mass spectrometric methods utilizing desorption of the moiety from the adsorbent and direct detection of the desorbed moiety. Moieties retained by the adsorbent after washing are adsorbed to the substrate. Moieties retained on the substrate are detected by desorption spectrometry.

The arrays of the invention are useful for surface-enhanced laser desorption/ionization, or SELDI. SELDI is described in U.S. Pat. No. 5,719,060 (Hutchens and Yip). SELDI is a method for desorption in which the moiety is presented to the energy stream on a surface that captures the moiety and, thereby, enhances moiety capture and/or desorption.

A plurality of detection means can be implemented in series to fully interrogate the moiety components and function associated with retentate at each location in the array.

Desorption detectors comprise means for desorbing the moiety from the adsorbent and means for directly detecting the desorbed moiety. That is, the desorption detector detects desorbed moiety without an intermediate step of capturing the moiety in another solid phase and subjecting it to subsequent analysis. Detection of a moiety normally will involve detection of signal strength. This, in turn, reflects the quantity of moiety adsorbed to the adsorbent.

The desorption detector also can include other elements, e.g., a means to accelerate the desorbed moiety toward the detector, and a means for determining the time-of-flight of the moiety from desorption to detection by the detector.

A preferred desorption detector is a laser desorption/ionization mass spectrometer, which is well known in the art. The mass spectrometer includes a port into which the substrate that carries the adsorbed moieties, e.g., a probe, is inserted. Striking the moiety with energy, such as laser energy desorbs the moiety. Striking the moiety with the laser results in desorption of the intact moiety into the flight tube and its ionization. The flight tube generally defines a vacuum space. Electrified plates in a portion of the vacuum tube create an electrical potential which accelerate the ionized moiety toward the detector. A clock measures the time of flight and the system electronics determines velocity of the moiety and converts this to mass. As any person skilled in the art understands, any of these elements can be combined with other elements described herein in the assembly of desorption detectors that employ various means of desorption, acceleration, detection, measurement of time, etc. An exemplary detector further includes a means for translating the surface so that any spot on the array is brought into line with the laser beam.

Arrays generated using the above teachings are highly reproducible, characterized by a signal to noise ratio of at least 2, 10, 20, 50, 100, 200, 500, 1000, 5000 or even more preferably 10,000.

As high-resolution, high-sensitivity datasets acquired using the arrays of the invention become available to the art, significant progress in the areas of diagnostics, therapeutics, drug development, biosensor development, and other related areas will occur. For example, disease markers can be identified and utilized for better confirmation of a disease condition or stage (see, U.S. Pat. Nos. 5,672,480; 5,599,677; 5,939,533; and 5,710,007). Subcellular toxicological information can be generated to better direct drug structure and activity correlation (see, Anderson, L., "Pharmaceutical Proteomics: Targets, Mechanism, and Function," paper presented at the IBC Proteomics conference, Coronado, Calif. (Jun. 11-12, 1998)). Subcellular toxicological information can also be utilized in a biological sensor device to predict the likely toxicological effect of chemical exposures and likely tolerable exposure thresholds (see, U.S. Pat. No. 5,811,231). Similar advantages accrue from datasets relevant to other biomolecules and bioactive agents (e.g., nucleic acids, saccharides, lipids, drugs, and the like).

Thus, described is a database that includes at least one set of data assay data. The data contained in the database is acquired using an array of the invention and/or a QD-labeled species either singly or in a library format. The database can be in substantially any form in which data can be maintained and transmitted, but is preferably an electronic database. The electronic database can be maintained on any electronic device allowing for the storage of and access to the database, such as a personal computer, but is preferably distributed on a wide area network, such as the World Wide Web.

The focus of the present section on databases, which include peptide sequence specificity data, is for clarity of illustration only. It will be apparent to those of skill in the art that similar databases can be assembled for any assay data acquired using an assay of the invention.

The compositions and methods described herein useful for identifying and/or quantitating the relative and/or absolute abundance of a variety of molecular and macromolecular species from a biological sample provide an abundance of information, which can be correlated with pathological conditions, predisposition to disease, drug testing, therapeutic monitoring, gene-disease causal linkages, identification of correlates of immunity and physiological status, among others. Although the data generated from the assays is suited for manual review and analysis prior data processing using highspeed computers is utilized.

An array of methods for indexing and retrieving biomolecular information is known in the art. For example, U.S. Pat. Nos. 6,023,659 and 5,966,712 disclose a relational database system for storing biomolecular sequence information in a manner that allows sequences to be catalogued and searched according to one or more protein function hierarchies.

Described is a computer database comprising a computer and software for storing in computer-retrievable form assay data records cross-tabulated, for example, with data specifying the source of the moiety-containing sample from which each sequence specificity record was obtained.

Also described is the storage and retrieval of a collection of moiety data in a computer data storage apparatus, which can include magnetic disks, optical disks, magneto-optical disks, DRAM, SRAM, SGRAM, SDRAM, RDRAM, DDR RAM, magnetic bubble memory devices, and other data storage devices, including CPU registers and on-CPU data storage arrays.

When the screenable moiety is a peptide or nucleic acid a method for identifying related peptide or nucleic acid sequences, comprising performing a computerized comparison between a peptide or nucleic acid sequence assay record stored in or retrieved from a computer storage device or database and at least one other sequence is described. The comparison can include a sequence analysis or comparison algorithm or computer program embodiment thereof (e.g., FASTA, TFASTA, GAP, BESTFIT) and/or the comparison may be of the relative amount of a peptide or nucleic acid sequence in a pool of sequences determined from a polypeptide or nucleic acid sample of a specimen.

Also described is a magnetic disk, such as an IBM-compatible (DOS, Windows, Windows95/98/2000, Windows NT, OS/2) or other format (e.g., Linux, SunOS, Solaris, AIX, SCO Unix, VMS, MV, Macintosh, etc.) floppy diskette or hard (fixed, Winchester) disk drive, comprising a bit pattern encoding data from an assay in a file format suitable for retrieval and processing in a computerized sequence analysis, comparison, or relative quantitation method.

Also described is a network, comprising a plurality of computing devices linked via a data link, such as an Ethernet cable (coax or 10BaseT), telephone line, ISDN line, wireless network, optical fiber, or other suitable signal tranmission medium, whereby at least one network device (e.g., computer, disk array, etc.) comprises a pattern of magnetic domains (e.g., magnetic disk) and/or charge domains (e.g., an array of DRAM cells) composing a bit pattern encoding data acquired from an assay.

Also described is a method for transmitting assay data that includes generating an electronic signal on an electronic communications device, such as a modem, ISDN terminal adapter, DSL, cable modem, ATM switch, or the like, wherein the signal includes (in native or encrypted format) a bit pattern encoding data from an assay or a database comprising a plurality of assay results.

Also described is a computer system for comparing a query moiety to a database containing an array of data structures, such as an assay result, and ranking database targets based on the degree of identity and gap weight to the moiety data. A central processor is preferably initialized to load and execute the computer program for alignment and/or comparison of the assay results. Data for a query moiety is entered into the central processor via an I/O device. Execution of the computer program results in the central processor retrieving the assay data from the data file, which comprises a binary description of an assay result.

The moiety data or record and the computer program can be transferred to secondary memory, which is typically random access memory (e.g., DRAM, SRAM, SGRAM, or SDRAM). Targets are ranked according to the degree of correspondence between a selected assay characteristic (e.g., binding to a selected binding functionality) and the same characteristic of the query moiety and results are output via an I/O device. For example, a central processor can be a conventional computer (e.g., Intel Pentium, PowerPC, Alpha, PA-8000, SPARC, MIPS 4400, MIPS 10000, VAX, etc.); a program can be a commercial or public domain molecular biology software package (e.g., UWGCG Sequence Analysis Software, Darwin); a data file can be an optical or magnetic disk, a data server, a memory device (e.g., DRAM, SRAM, SGRAM, SDRAM, EPROM, bubble memory, flash memory, etc.); an I/O device can be a terminal comprising a video display and a keyboard, a modem, an ISDN terminal adapter, an Ethernet port, a punched card reader, a magnetic strip reader, or other suitable I/O device.

Also described is the use of a computer system, such as that described above, which comprises: (1) a computer; (2) a stored bit pattern encoding a collection of peptide sequence specificity records obtained by the methods described which may be stored in the computer; (3) a comparison moiety, such as a query moiety; and (4) a program for alignment and comparison, typically with rank-ordering of comparison results on the basis of computed similarity values.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

### MATERIALS AND EXPERIMENTAL METHODS

### Reaction Conditions and Reagents:

All reactions were conducted under a nitrogen atmosphere and in darkness.

Dichloromethane, pyridine, and ethyl ether were purchased anhydrous from Aldrich; triethylamine (TEA) was distilled from CaH₂; and ω-amino-α-carboxyl PEG (MW 3,400; Shearwater Polymers, Inc.) was dried by azeotropic distillation with benzene.

### Atomic Force Microscopy (AFM) Measurements:

AFM scans in tapping mode were performed on a Silicon (100) wafers coated with 100 nm of SiO₂ (obtained from MEMC electronic materials, Inc.) using a multimode Nanoscope IIIa VEECO-DI (Santa Barbara, CA) and 280-400 kHz resonance frequency silicon probes (Olympus OTESP). AFM images were scanned in air under ambient laboratory conditions.

### X-ray Reflectometry (XRR) Measurements;

XRR measurements were conducted using X23B beam line of the Brookhaven National Laboratories Synchrotron, in a four-circle Huber diffractometer in specular reflection mode. The reflected intensity was measured as a function of the scattering vector component *q_{z}*=(4π/λ)sinθ, perpendicular to the reflecting surface. Incident X-ray energy was 10 KeV (λ=1.240Å), and 0.4 mm vertical, 2 mm horizontal beam size. Samples were kept under slight Helium overpressure during the measurements in order to reduce the background scattering from the ambient gas and radiation damage. Measurements were performed at room temperature. Off-specular background was measured and subtracted from specular counts.

### Spectroscopic Ellipsometry and Contact Angle Measurements:

Film thickness was measured using variable angle spectroscopic ellipsometer WVASEE32 (J. A. Woollam Co. Inc) instrument. Measurements were conducted at 70 ° incidence angle and spectrum range of 390-900nm. The assumed index of refraction for PEG was 1.460. Water-substrate contact angles were measured in air using Rame-Hart Automated Contact Angle Goniometer.

### Preparation of Cy3-tagged DNA:

Biotinilated dsDNA is tagged with cy3 fluorescent dye according to manufacturer's instructions (Mirus/*Label* IT@ Cy^{™}3 Labeling Kit).

### Cell-free protein synthesis:

Cell-free coupled transcription-translation reactions were carried out using wheat germ cell extracts (TNT, Promega) according to the manufacturer protocol.

### EXAMPLE 1

### Preparation of all exemplary trifunctional compound for integrating screenable moieties onto a solid substrate

In a search for a simple-to-use, single step and inexpensive technology that integrates all previous successful approaches in one molecule, namely, a self-assembled dense monolayer that resists non-specific adsorption and supports light-directed, sub-micron immobilization of a variety of biomolecules via peptide bonds, a general chimera molecule, in which three functional modules are chemically linked together, has been designed. An exemplary such chimera molecule, also referred to herein as "daisy", has been successfully synthesized, as shown in Figure 1 and is further detailed hereinunder. As an exemplary starting material and backbone of the new chimera molecule, a bi-functional PEG (3400 MW) with a carboxyl and an amine at its opposite ends was selected. Amino-propyl-triethoxy-silane (APTS) was attached to the carboxyl end serving as an anchoring reactive group for grafting to silicon or glass substrates. A protecting group, 6-nitroveratryl chloroformate (6NVOC), was attached to the PEG amine, which thus becomes available for specific chemical coupling only upon UV-de-protection.

The two-steps synthesis was performed and quality-controlled in bulk phase solution, thus alleviating any step-wise synthesis on the surface. This fact improves the signal-to-noise ratio that often deteriorates with successive *in situ* step-wise attachments, and simplifies the approach. A PEG molecule with a molecular weight of 3400 was selected so as to minimize non specific absorptions. Indeed, the ability of a PEG layer to prevent non-specific absorption depends both on polymer molecular weight and grafting density. Both low and high molecular weight PEGs are able to prevent adsorption. However, longer molecules seem to be more effective, as long as their grafting density is high enough to allow the polymer chains to half overlap each other [17, 27].

***Synthesis of N_{ω}-Nvoc-amine-α-carboxyl PEG (Compound 1,*** ***Figure 1******):*** A solution of ω-amino-α-carboxyl PEG (500 mg, 0.15 mmol) in CH₂Cl₂ (8 ml) was treated with 4-DMAP (20 mg) and pyridine (196 mg, 2.48 mmol). The mixture was stirred for 5 minutes and a solution of 6-nitroveratryl chloroformate (200 mg, 0.73 mmol) in CH₂Cl₂ (4 ml) was added. The reaction vessel was flushed with N₂, sealed, and stirred for 4 days at room temperature. The solvent was evaporated, the residue was dissolved in a minimum amount of CH₂Cl₂ and the desired Compound **1** was crystallized by slow addition of cold ethyl ether (525 mg, 98 %).

¹H NMR (250 MHz, CDCl₃): δ = 3.63 (m, 278 H), 3.95 (s, 3 H), 3.98 (s, 3 H), 5.52 (d, 2 H, *J* = 5.6), 7.02 (m, 1 H), 7.71 (m, 1 H) ppm.

***Synthesis of N_{ω}-Nvoc-amine-N_{α}-[3-(triethoxysilyl)propyl]-carboxamide PEG (Compound 2, "Daisy",*** ***Figure 1******):*** A solution of Compound **1** (500 mg, 0.14 mmol) in CH₂Cl₂ (20 ml) was treated with 1,3-dicyclohexylcarbodiimide (90 mg, 0.44 mmol) and N-hydroxysuccinimide (50 mg, 0.44 mmol). The mixture was stirred overnight at room temperature and the precipitated urea was removed by filtration. The filtrate was treated with 3-aminopropyltriethoxysilane (97 mg, 0.44 mmol) followed by TEA (45 mg, 0.44 mmol) and the resulting mixture was stirred overnight at room temperature. The solvent was evaporated and the residue was dissolved in a minimum amount of CH₂Cl₂ and precipitated with cold dry diethylether. Two hundreds mg were recovered and the yield of derivatization was 60 %.

¹H NMR (250 MHz, CDCl₃): δ = 1.22 (t, 5H, *J* = 7), 3.64 (m, 278 H), 3.96 (s, 3 H), 3.99 (s, 3 H), 5.53 (d, 2 H, J = 7.6), 7.04 (m, 1 H), 7.71 (m, 1H) ppm.

Aliquots of Compound **2** can be stored in powder form under dry conditions to avoid self-polymerization of the reactive silanes.

### EXAMPLE 2

### Formation of UV-activatable Films

UV-activatable films (monolayers) were formed by attaching the chimera molecule (e.g., Compound **2**) to a silica-coated silicon surface. The chimera molecule is preferably designed such that upon incubation with a silica-coated surface in an appropriate solvent, the molecule interacts with the SiO₂ surface hydroxyl groups, so as to spontaneously form a monolayer on the surface.

Thus, Compound **2** was reacted with silicon (100) wafers coated with 100 nm of SiO₂ (obtained from MEMC electronic materials, Inc.), as shown in Figure 2, by incubation in 100 µl toluene at concentration of 0.22 mg/ml on a 1x1cm² wafer. A "daisy" monolayer film (Compound **3**, Figure 2) forms spontaneously on a silicon dioxide surface within few minutes of incubation in toluene.

Figure 3 presents AFM images of silica-coated (100 nm thick SiO₂ layer) silicon wafers before (top) and after (bottom) incubation with Compound **2**. As is shown in Figure 3, atomic force microscope scans of daisy films on 100 nm SiO₂-coated silicon wafers substrates (Compound **3**, Figure 2) reveal smooth topography, a coarse grain of nanometric SiO₂ roughness.

X-ray reflectometry (XRR) was used to measure "daisy" film thickness, roughness and molecular footprinting, as described in the experimental methods section hereinabove. Three SiO₂-coated silicon wafers were incubated with daisy for 1 minute, 10minutes and 60minutes. The results indicated that a thickness of 16.0 ± 0.3 Å with roughness of 4.0 ± 0.1 Å, and area per molecule (footprint) of 293 ± 10 (Å)² were obtained, independently of the incubation time.

The film thickness was confirmed by ellipsometry, as described hereinabove. The contact angle with water of a newly formed daisy film on a SiO₂ surface was about 50°, consistent with a wetting interface.

These data are consistent with a monolayer of daisy in which the polymer is in a mushroom like conformation [19]. The calculated grafting density (190 ng/cm²) is well above the levels required to prevent non specific absorption of proteins [27].

### EXAMPLE 3

### Activation of Daisy Films

The "daisy" film (Compound **3**) can be readily subjected to UV-irradiation, to thereby lithographically de-protect the amine surface groups of the chimera (Figure 4, Compound **4**).

Thus, shortly after formation, the "daisy" film was used for lithographical de-protection with the 365nm I-line of a mercury arc lamp used with a standard fluorescent optical microscope. The required pattern of amines on the daisy film was printed on transparency paper that serves as a mask placed at the field stop of the fluorescent light path. The mask was de-magnified and projected on the daisy film by the microscope objective lens [28], to produce rapid patterning molecules at sub-micron resolution, while alleviating the need for expensive contact-mode mask lithography.

Activated "daisy-coated" slides (Compound **4**, Figure 4) can be prepared in advance and stored in darkness for future use.

### EXAMPLE 4

### Integration of Biomolecules on the Activated Films

Simple and efficient integration of various biomolecules can thereafter be performed on the exposed amino groups of the activated film.

As a representative example, integration of the biotin-avidin high affinity binding system was demonstrated, as is detailed hereinbelow.

### Preparation of biotinylated films:

As shown in Figure 4, the de-protected (photogenerated) amines in Compound 4 were reacted with a biotin derivative (biotin-*n*-hydroxysuccinimide, NHS-biotin, Sigma) in Borate buffer 270 mM, pH 8.35, for a couple of hours, to thereby form amide bonds between the surface free amino groups of the activated film and the biotin carboxylic groups. The resulting biotinylated films (Compound **5**) were then extensively washed with NaCl 1M, followed by Tris (Tris 20 mM, NaCl 150 mM).

These biotinylated films can serve as an efficient platform for integrating a plethora of biomolecules to the array as is, with no need for subsequent chemistry. Alternatively, biomolecules can be attached to the biotinylated film in steps, as is exemplified hereinunder.

### Conjugation of Avidin to Biotinylated films:

The 2D-patterned biotinylated films described above (Compound **5**, Figure 4) were visualized with fluorescein-tagged avidin.

To this end, avidin protein labeled with fluorescein was reacted with surface biotin molecules of the biotinylated film 9Compound **5**, prepared as described above, and incubated in Tris buffer at 3.3 µg/ml concentration, for one hour at room temperature, followed by rinsing in Tris, to thereby produce Daisy films having a plurality of surface avidin molecules attached thereto.

Figure 5 presents a 2D pattern of avidin protein labeled with fluorescein, generated with a printed `AVIDIN' photo-mask placed at the field stop of a microscope and clearly show that avidin protein readily binds tightly to the 2D patterned biotinylated films.

### Patterning Calibration:

Calibrating of the 365nm flux, required for successful patterning, was done by varying the intensity and duration of the UV light pulse through the field stop and a 60x objective lens, and using fluorescein-tagged avidin, prepared as described hereinabove, to probe immobilization.

A "daisy-coated" glass slide (Compound **3**) was exposed through an octagonal mask at varying duration (5 seconds, 30 seconds, 2 minutes and 10 minutes) and varying UV light intensity (0.44 mW/cm², 4.4 mW/cm², 18.7 mW/cm² and 70 mw/cm²). Fluorescein-tagged avidin was thereafter conjugated to the patterned film, via biotinylation, as described hereinabove. The results are presented in Figures 6a and 6b.

As is shown in Figure 6a, at low UV flux only partial de-protection occurs while excess flux likely damages the film, resulting in a halo around the exposed octagon.

The signal-to-noise ratio (S/N) for patterning DNA molecules on Daisy-coated chips was tested. 2000 base pair-double stranded DNA labeled with one biotin at the 5'-end was conjugated to avidin in advance prior to incubation with the surface, one DNA per avidin protein. The avidin used was fluorescein-labeled for quantification on the surface.

A single chip was divided into nine equal regions, each 3 mm in diameter. The regions were exposed uniformly at increasing photolithography 365 nm UV flux from 0 to 2.5 Joule/cm² in order to increase gene density. Biotin-NHS was reacted with the de-protected surface amines, as described. The avidin-conjugated DNA molecules were incubated with the chip, which were then thoroughly washed to remove excess non-specific adsorption. The fluorescence of the surface bound molecules was measured as a function of UV flux used for patterning, revealing a monotonous increasing density of DNA with UV flux with a S/N of 190 between 0 to maximum UV flux used.

Working in the region where the Daisy interface responds linearly with UV flux allows the control of molecular density by single-step lithography. This can be effected by transferring a gray-scale image to a photographic sensitivity film, which is then used as a mask for lithography. Figure 6c shows biotinylated-dsDNA bound to fluorescein-tagged avidin in a grayscale mode.

### Conjugation of neutra-Avidin and DNA to Biotinylated films:

Neutra-avidin (Pierce, at a concentration of 3.3 µg/ml) was reacted with surface biotin molecules of biotinylated Daisy films, prepared as described above, and incubated in Tris buffer at 3.3 µg/ml concentration, for one hour at room temperature, followed by rinsing in Tris buffer, to thereby produce "daisy" film having a plurality of surface neutral-avidin molecules attached thereto.

Subsequently, biotinylated double-stranded DNA (dsDNA), 10 nM in double-distilled water (DDW) or a buffered solution (e.g., Tris, Phosphate,), was incubated for one hour to bind to the surface neutra-avidin pattern.

Alternatively, 50 nM biotinilated dsDNA in Tris were coupled to neutra-avidin prior to immobilization (10 to 1 molar ratio of avidin to DNA), filtered, using 100 kD filters (Millipore/Microcon filters), and than incubated on biotinylated "daisy" films for 1 hour, so as to couple to surface biotins.

***Conjugation of Cy3-tagged DNA to patterned Biotinylated films:*** Biotinilated dsDNA, 2000 base pairs long, tagged with cy3 dye, as described in the experimental methods section above, was reacted with neutra-avidin prior to immobilization, as described hereinabove, was thereafter conjugated to a patterned biotinylated film, generated with a printed 'dsDNA' photo-mask placed at the field stop of a microscope.

Figure 7 presents the localization of the biotinylated double-stranded DNA tagged with cy3-dye and clearly show only minimal non-specific adsorption of such long (about 2000 base pairs) DNA molecules that would otherwise tend to adsorb onto a less inert interface.

### EXAMPLE 5

### Functionality of biomolecules integrated in the Films - on-chip protein synthesis

Functionality of biomolecules integrated in the films of the present invention was demonstrated by performing on-chip cell-free protein synthesis.

To this end, biotinylated double-stranded DNA transcriptional units were prepared in which firefly *luciferase* gene was cloned under T7 RNA polymerase promoter [29]. Using a ~50x50µm² octagonal mask, chips were patterned with 2, 4, 8 and 16 octagons of the Biotinylated dsDNA, respectively, a corresponding to as little as about 1-10% of total surface area.

Coupled transcription/translation reactions using the cell-free T7/wheat germ system were incubated with each chip and the kinetics of on-chip luciferase protein synthesis was measured by a standard luminescence assay. Luminescence from 2 µl of the luciferase cell-free synthesis reaction mixture from the chip added to 18 µl luciferase assay reagent (Roche) was measured using a photo-multiplier tube (H7155, Hamamatsu) and a counter (53131A 225 MHz, Agilent). The results, presented in Figure 8a, show that the total protein yield from surface-integrated genes is as high as from gene expressed in solution, at saturating concentration of 1nM. Furthermore, as depicted in Figure 8b, the total yield increases proportionally to the number of octagon patterns, as expected [29] and consistent with minimal non-specific DNA adsorption.

### EXAMLPE 6

### The effect of surface-coverage on oil-chip protein synthesis

The functionality of surface immobilized dsDNA molecules generated according to the teachings of the present invention was substantiated as described in Example 5, above. The effect of surface coverage and density was then tested to assess miniaturization potential of chips generated according to the teachings of the present invention.

As in Example 5 above, on-chip protein synthesis was carried out using cDNA coding for firefly *luciferase* reporter gene under T7 RNA polymerase promoter, with avidin-conjugated biotinylated DNA integrated on chips as described above.

Luciferase gene was patterned at high density on nine large surfaces, each 3 mm in diameter, on a single chip. Each pattern consisted of a basic 10x10 µm² repeat unit arranged in an extended two-dimensional array at fixed distances, ranging from 400 to 0 µm, thus covering from 0.06% to 100% surface coverage (Figure 9a). Coupled transcription/translation reactions using the cell-free T7/wheat-germ system were incubated with each array pattern on the chip and Luciferase protein synthesis yield was measured by a standard luminescence assay.

Figure 9a shows that similarly to *in vitro* transcription/translation reactions performed in bulk solution where protein yield is proportional to DNA concentration, protein yields of the present surfaces were proportional to surface coverage. At low surface coverage with 400 µm between repeat units there is only a minute residual protein synthesis from nonspecifically adsorbed avidin-conjugated DNA with 250-fold lower yield compared to full surface coverage.

The effect of gene surface density on protein yield was then tested.

A single chip was divided into nine equal regions, each 3 mm in diameter. The regions were exposed uniformly at increasing photolithography UV flux to increase reporter gene density. The density of the patterned molecules was tuned by calibration of the 365 nm flux. This was effected by varying the intensity and duration of the UV light pulse. The *luciferase* gene was integrated on the entire chip for subsequent protein synthesis measurement.

Figure 9b clearly shows that dense genes produce 700-fold more proteins than diluted ones in the UV flux range probed, demonstrating a wide dynamic range of protein synthesis on the chip. Any residual protein synthesis resulted from unpatterned nonspecifically adsorbed avidin-conjugated genes. Basically, this residual activity can come from nonspecific adsorption of DNA molecules alone without the conjugated avidin, or from avidin binding to biotin molecules randomly distributed on the surface. Figure 9b clearly shows contribution of protein synthesis from genes incubated on the surface without Avidin conjugated to them. The signal is ~5000 fold lower than for avidin-conjugated genes at the highest density. This result implies that residual protein synthesis activity on daisy chips is not due to non-specifically adsorbed DNA molecules. It is likely due to avidin binding to biotin-NHS molecules integrated to a small fraction of un-protected amines left on daisy molecules during its initial chemical synthesis. The synthesis of daisy can, therefore, slightly be improved to essentially eliminate all residual non-specific protein activity on the chip.

### EXAMPLE 7

### Functionality of biomolecules integrated in the Films - on-chip protein binding

Functionality of biomolecules integrated in the films of the present invention was demonstrated by performing on-chip protein binding assay.

Daisy-coated chips were tested for trapping proteins from crude extracts without prior purification. Micro-traps for tagged proteins synthesized *in vitro* were designed based on the interaction between an antibody and the HA peptide tag (amino acid sequence YPYDVPDYA (SEQ ID NO: 1), included in a commercially available plasmid (Roche, pIVEX 2.5d and 2.6d).

A fusion between the HA peptide tag and the *enhanced green fluorescent protein (egfp)* gene was sub-cloned under the T7 promoter for synthesis using a cell-free transcription/translation system. An expression vector for C-terminal (*T7-egfp-HA*) fusion was prepared.

Two DNA plasmids were constructed encoding N-terminal and C-terminal EGFP and HA fusions under the T7 promoter using the commercial vectors pIVEX 2.5d and pIVEX 2.6d for cell-free expression in *E. coli* extract system (RTS500, Roche). The pIVEX 2.5d_egfp and pIVEX_2.6d_egfp were prepared by restriction enzyme digestion of the pIVEX 2.5d and pIVEX 2.6d plasmids at unique NcoI and SmaI sites. The *egfp* gene was amplified in a PCR reaction from T7-egfp plasmid^{[27]}, using primers with extensions of NcoI site at the N-terminus and SmaI at the C-terminal. Primers for amplification of *egfp* without stop codon (TAA) and with extension of NcoI site at the N-terminal and SmaI at the C-terminal:

A monoclonal biotinylated antibody to the HA peptide tag was patterned on-chip using a two-dimensional array of 10x10 µm² squares following avidin localization, as described above (see scheme of Figure 10). Cell-free protein synthesis of the HA-tagged EGFP was first carried out in solution using the expression vector *T7-egfp-HA*. Subsequently, the synthesis reaction in the cell extract was incubated with the protein trap chip.

With about 10-100 mg/ml endogenous proteins in the cell extracts significant debris is expected to be adsorbed on the chip and mask any predefined patterns. H, a pattern of tagged-EGFP was retained after washing off, thus demonstrating both specific protein localization and resistance to nonspecific adsorption from crude extracts.

### REFERENCES CITED BY NUMERALS

### (ADDITIONAL REFERENCED ARE CITED WITHIN THE TEXT)

1. Schena, M., Shalon, D., Davis, R.W. & Brown, P.O. Quantitative Monitoring Of Gene-Expression Patterns With A Complementary-Dna Microarray. Science 270, 467-470 (1995).
2. Xiao, S., Textor, M., Spencer, N. & Sigrist, H. Covalent attachment of cell-adhesive, (Arg-Gly-Asp)-containing peptides to titanium surfaces. LANGMUIR 14, 5507-5516 (1998).
3. Shin, D. et al. Protein patterning by maskless photolithography on hydrophilic polymer-grafted surface. BIOSENS BIOELECTRON 19, 485-494 (2003).
4. BASCOM, W. STRUCTURE OF SILANE ADHESION PROMOTER FILMS ON GLASS AND METAL SURFACES. MACROMOLECULS 5, 792-798 (1972).
5. Boerio, F., Armogan, L. & Cheng, S. THE STRUCTUR OF g-AMINOPROPYLTRIETHOXYSILANE FILMS ON IRON MIRRORS. JOURNAL OF COLLOID AND INTERFACE SCIENCE 73, 416-424 (1980).
6. PLUEDDEMANN, E. REMINISCING ON SILANE COUPLING AGENTS. J ADHES SCI TECHNOL 5, 261-277 (1991).
7. Wagner, P., Hegner, M., Kernen, P., Zaugg, F. & Semenza, G. Covalent immobilization of native biomolecules onto Au(111) via N-hydroxysuccinimide ester functionalized self-assembled monolayers for scanning probe microscopy. BIOPHYS J 70, 2052-2066 (1996).
8. Wagner, P., Zaugg, F., Kernen, P., Hegner, M. & Semenza, G. omega-Functionalized self-assembled monolayers chemisorbed on ultraflat Au(111) surfaces for biological scanning probe microscopy in aqueous buffers. J VAC SCI TECHNOL B 14, 1466-1471 (1996).
9. Zaugg, F. et al. Microstructured bioreactive surfaces: covalent immobilization of proteins on Au(111)/silicon via aminoreactive alkanethiolate self-assembled monolayers. J MATER SCI-MATER M 10, 255-263 (1999).
10. Gauvreau, V. et al. Engineering surfaces for bioconjugation: Developing strategies and quantifying the extent of the reactions. BIOCONJUGATE CHEM 15, 1146-1156 (2004*).*
11. Kroger, D., Liley, M., Schiweck, W., Skerra, A. & Vogel, H. Immobilization of histidine-tagged proteins on gold surfaces using chelator thioalkanes. BIOSENS BIOELECTRON 14, 155-161 (1999).
12. Sigal, G., Bamdad, C., Barberis, A., Strominger, J. & Whitesides, G. A self-assembled monolayer for the binding and study of histidine tagged proteins by surface plasmon resonance. ANAL CHEM 68, 490-497 (1996).
13. WILCHEK, M. & BAYER, E. THE AVIDIN BIOTIN COMPLEX IN BIOANALYTICAL APPLICATIONS. ANAL BIOCHEM 171, 1-32 (1988).
14. PALEGROSDEMANGE, C., SIMON, E., PRIME, K. & WHITESIDES, G. FORMATION OF SELF-ASSEMBLED MONOLAYERS BY CHEMISORPTION OF DERIVATIVES OF OLIGO(ETHYLENE GLYCOL) OF STRUCTURE HS(CH2)11(OCH2CH2)META-OH ON GOLD. J AM CHEM SOC 113, 12-20 (1991).
15. JEON, S. & ANDRADE, J. PROTEIN SURFACE INTERACTIONS IN THE PRESENCE OF POLYETHYLENE OXIDE.2. EFFECT OF PROTEIN SIZE. JOURNAL OF COLLOID AND INTERFACE SCIENCE 142, 159-166 (1991).
16. JEON, S., LEE, J., ANDRADE, J. & DEGENNES, P. PROTEIN SURFACE INTERACTIONS IN THE PRESENCE OF POLYETHYLENE OXIDE.1. SIMPLIFIED THEORY. JOURNAL OF COLLOID AND INTERFACE SCIENCE 142,149-158 (1991).
17. Du, H., Chandaroy, P. & Hui, S. Grafted poly-(ethylene glycol) on lipid surfaces inhibits protein adsorption and cell adhesion. BBA-BIOMEMBRANES 1326, 236-248 (1997).
18. Lu, H., Campbell, C. & Castner, D. Attachment of functionalized poly(ethylene glycol) films to gold surfaces. LANGMUIR 16, 1711-1718 (2000).
19. Yang, Z., Galloway, J. & Yu, H. Protein interactions with poly(ethylene glycol) self-assembled monolayers on glass substrates: Diffusion and adsorption. LANGMUIR 15, 8405-8411 (1999).
20. Harder, P., Grunze, M., Dahint, R., Whitesides, G. & Laibinis, P. Molecular conformation in oligo(ethylene glycol)-terminated self-assembled monolayers on gold and silver surfaces determines their ability to resist protein adsorption. J PHYS CHEM B 102, 426-436 (1998).
21. Metzger, S., Natesan, M., Yanavich, C., Schneider, J. & Lee, G. Development and characterization of surface chemistries for microfabricated biosensors. J VAC SCI TECHNOL A 17, 2623-2628 (1999).
22. Ruiz-Taylor, L. et al. Monolayers of derivatized poly(L-lysine)-grafted poly(ethylene glycol) on metal oxides as a class of biomolecular interfaces. P NATL ACAD SCI USA 98, 852-857 (2001).
23. FODOR, S. et al. LIGHT-DIRECTED, SPATIALLY ADDRESSABLE PARALLEL CHEMICAL SYNTHESIS. SCIENCE 251, 767-773 (1991).
24. Kumar, P., Agarwal, S. & Gupta, K. N-(3-trifluoroethanesulfonyloxypropyl)anthraquinone-2-carboxamide: A new heterobifunctional reagent for immobilization of biomolecules on a variety of polymer surfaces. BIOCONJUGATE CHEM 15, 7-11 (2004).
25. Nivens, D. & Conrad, D. Photoactive poly(ethylene glycol) organosilane films for site-specific protein immobilization. LANGMUIR 18, 499-504 (2002).
26. McGall, G.H. & Christians, F.C. High-density genechip oligonucleotide probe arrays. Adv Biochem Eng Biotechnol 77, 21-42 (2002).
27. Sofia, S., Premnath, V. & Merrill, E. Poly(ethylene oxide) grafted to silicon surfaces: Grafting density and protein adsorption. MACROMOLECULES 31, 5059-5070 (1998).
28. Love, J., Wolfe, D., Jacobs, H. & Whitesides, G. Microscope projection photolithography for rapid prototyping of masters with micron-scale features for use in soft lithography. LANGMUIR 17, 6005-6012 (2001).
29. Noireaux, V., Bar-Ziv, R. & Libchaber, A. Principles of cell-free genetic circuit assembly. P NATL ACAD SCI USA 100, 12672-12677 (2003).
30. Oleinikov, A. et al. Self-assembling protein arrays using electronic semiconductor microchips and in vitro translation. J PROTEOME RES 2, 313-319 (2003).
31. Ramachandran, N. et al. Self-assembling protein microarrays. SCIENCE 305, 86-90 (2004).
32. He, M. & Taussig, M. DiscernArray (TM) technology: a cell-free method for the generation of protein arrays from PCR DNA. J IMMUNOL METHODS 274, 265-270 (2003).
33. Tabuchi, M., Hino, M., Shinohara, Y. & Baba, Y. Cell-free protein synthesis on a microchip. PROTEOMICS 2, 430-435 (2002).

## Claims

1. A compound comprising:
a functionalized group capable of binding to a solid substrate, said functionalized group comprising at least one reactive silyl group;
a photoactivatable group capable of generating a reactive group upon exposure to light, said reactive group being capable of binding a screenable moiety; and
a polyethylene glycol (PEG) polymer capable of forming a monolayer on said solid substrate, said polymer having said functionalized group and said photoactivatable group attached thereto;
said polyethylene glycol having a molecular weight that ranges from 2000 grams/mol to 5000 grams/mol,
the compound is being capable of forming on said solid substrate a monolayer terminating with said photoactivatable group, upon contacting said solid substrate in a one-step reaction.

2. The compound of claim 1, having a general formula I:
**X- L-Y** Formula I
wherein:
X is said functionalized group comprising said at least one reactive silyl group and being capable of binding to said solid substrate;
L is said PEG polymer capable of forming said monolayer onto said solid substrate; and
Y is said photoactivatable group capable of generating said reactive group upon exposure to said light.

3. The compound of any of claims 1 and 2, wherein said reactive silyl group is trialkoxysilane.

4. The compound of claim 1, wherein said polymer comprises a substituted or unsubstituted polyethylene glycol (PEG).

5. The compound of claim 1, wherein said reactive group generated upon exposure of said photoactivatable group to said light is selected from the group consisting of amine, hydroxy, thiohydroxy, halo, alkoxy, thioalkoxy, aryloxy, thioaryloxy, carboxylate, phosphate, phosphonate, sulfate and sulfonate.

6. The compound of claim 1, wherein said photoactivatable group comprises a carbamate.

7. The compound of claim 6, wherein said reactive group generated upon exposure of said photoactivatable group to said light is amine.

8. The compound of claim 1, wherein said screenable moiety is selected from the group consisting of a biological moiety and chemical moiety.

9. A process of preparing the compound of any of claims 1-8, the process comprising:
providing a polyethylene glycol (PEG) polymer having a molecular weight that ranges from 2000 grams/mol to 5000 grams/mol and containing a first reactive group and a second reactive group, wherein said first reactive group is capable of reacting with a first compound, said first compound containing said functionalized group which comprises said at least one silyl group, and said second reactive group is capable of reacting with a second compound to thereby form said photoactivatable group;
reacting said polymer with said first compound; and
reacting said polymer with said second compound.

10. The process of claim 9, wherein said first compound further comprises a third reactive group, said third reactive group being capable of reacting with said first reactive group of said PEG polymer.

11. The process of claim 10, wherein said first reactive group and said third reactive form a linking moiety, said linking moiety linking said functionalized group to said PEG polymer.

12. The process of claim 9, wherein said second compound comprises a residue of said photoactivatable group being linked to a forth reactive group.

13. The process of claim 12, wherein said forth reactive is capable of reacting with said second reactive group.

14. The process of claim 9, wherein said polymer is a ω-amino-ω-carboxyl PEG having a molecular weight in the range from 2000 to 5000 grams/mol.

15. A process of attaching a screenable moiety to at least one pre-selected area of a solid substrate, the process comprising:
providing a solid substrate having at least one functional group on a surface thereof;
providing a compound having a functionalized group capable of binding to said solid substrate, said functionalized group comprising at least one reactive silyl group, a photoactivatable group capable of generating a reactive group upon exposure to a light source, said reactive group being capable of binding said screenable moiety, and a polyethylene glycol (PEG) polymer capable of forming a monolayer on said solid substrate and having said functionalized group and said photoactivatable group attached thereto, said PEG polymer having a molecular weight that ranges from 2000 grams/mol to 5000 grams/mol;
contacting said compound with said solid substrate, in a one-step reaction, by reacting said functionalized group with said functional group on said surface of the solid substrate, to thereby provide a solid substrate having a plurality of monolayers of said polymer attached thereto, wherein each of said monolayers terminates with said photoactivatable group;
exposing said at least one pre-selected area of said solid substrate having said monolayer of said polymer attached thereto to a light, to thereby generate said reactive group from said photoactivatable group at said pre-selected area; and
binding said screenable moiety to said reactive group, thereby attaching the screenable moiety to the pre-selected area of the solid support.

16. The process of claim 15, wherein said binding said screenable moiety to said reactive group comprises reacting said reactive group with said screenable moiety.

17. The process of claim 15, wherein said binding said screenable moiety to said reactive group comprises:
providing a mediating moiety capable of binding to said screenable moiety and to said reactive group; and
binding said screenable moiety to said reactive group via said mediating moiety.

18. The process of claim 17, wherein said mediating moiety comprises an affinity pair.

19. The process of claim 17, wherein said mediating moiety comprises a mediating agent.

20. A process of preparing an array of screenable moieties, the process comprising:
providing a solid substrate having a plurality of functional groups on a surface thereof;
providing a compound having a functionalized group capable of binding to said solid substrate, said functionalized group comprising at least one reactive silyl group, a photoactivatable group capable of generating a reactive group upon exposure to a light source, said reactive group being capable of binding a screenable moiety; and a polyethylene glycol (PEG) polymer capable of forming a monolayer on said solid substrate and having said functionalized group and said photoactivatable group attached thereto, said PEG polymer has a molecular weight that ranges from 2000 grams/mol to 5000 grams/mol;
contacting said compound to said solid substrate by reacting said functionalized group with said functional groups on said surface, to thereby provide, in a one-step reaction, a solid substrate having a plurality of monolayers of said polymer attached thereto, wherein each of said monolayers terminates with said photoactivatable group;
exposing a first pre-selected area of said solid substrate having said monolayer of said polymer attached thereto to a light, to thereby generate a reactive group from said photoactivatable group at said first pre-selected area; and
binding a screenable moiety to said reactive group at said first pre-selected area;
exposing a second pre-selected area of said solid substrate having said monolayer of said polymer attached thereto to a light, to thereby generate a reactive group from said photoactivatable group at said second pre-selected area;
binding a second screenable moiety to said reactive group at said second pre-selected area;
consecutively exposing an Nth pre-selected area of said solid substrate having said monolayer of said polymer attached thereto to a light, to thereby generate a reactive group at said Nth pre-selected area; and
binding an Nth screenable moiety to said reactive group at said Nth pre-selected area, thereby providing an array of screenable moieties.

21. The process of claim 20, wherein said binding said screenable moiety to said reactive group comprises:
providing a mediating moiety capable of binding to said screenable moiety and to said reactive group; and
binding said screenable moiety to said reactive group via said mediating moiety.

22. The process of claim 20, wherein said pre-selected area of said solid substrate ranges between 1x10⁻² cm² to 1x10⁻² cm² and 1x10⁻⁵ cm² to 1x10⁻⁵ cm².

23. The process of claim 20, wherein said N is in a range of from 2 to 2000.

24. An array of screenable moieties, prepared by the process of claim 20.

25. An array of screenable moieties, comprising:
a solid substrate having a plurality of monolayers of a polyethylene glycol (PEG) polymer applied thereon, said PEG polymer has a molecular weight that ranges from 2000 grams/mol to 5000 grams/mol and having a functionalized group and a photoactivatable group attached thereto, said functionalized group being capable of binding to said solid substrate, and comprising at least one reactive silyl group, said photoactivatable group being capable of generating a reactive group upon exposure to a light source, said reactive group being capable of binding said screenable moiety, wherein each of said plurality of monolayers of said PEG polymer is applied on said solid substrate, in a one-step reaction, by reacting a functionalized group attached to said polymer with a functional group on a surface of said solid substrate, said functionalized group comprising at least one reactive silyl group; and a plurality of screenable moieties being bound to said plurality of monolayers at pre-selected areas of said solid substrate, such that each of said screenable moieties is bound to each of said monolayers at a pre-selected area of said solid substrate upon exposure to light of a plurality of monolayers of said PEG polymer in which each monolayer terminates with a photoactivatable group, at said pre-selected area of said solid substrate, wherein said exposure to light generates a reactive group which binds said screenable moiety.

26. The array of claim 25, wherein at least a portion of said screenable moieties is bound directly to said plurality of monolayers of said PEG polymer.

27. The array of claim 25, wherein at least a portion of said screenable moieties is bound to a mediating moiety, whereas said mediating moiety is bound to said plurality of monolayers of said PEG polymer.

28. The array of claim 25, wherein said exposure to light of a photoactivatable group that is attached to said monolayer generates a reactive group at said pre-selected area of said solid substrate.

29. The array of claim 25, wherein said screenable moieties are selected from the group consisting of biological moieties and chemical moieties.

30. The array of claim 25, wherein each of said pre-selected areas of said solid substrate ranges between 1x10⁻² cm² to 1x10⁻² cm² and 1x10⁻⁵ cm² to 1x10⁻⁵ cm².

31. The array of claim 25, comprising from 1 to 20000 screenable moieties.

32. The array of claim 25, wherein the array is **characterized by** a signal-to-noise ratio of at least 200.

33. The array of claim 25, wherein said screenable moieties are bound to said solid substrate at a submicron resolution.

34. A solid substrate having the compound of claim 1 attached thereon.

## Patentansprüche

1. Verbindung, umfassend:
eine funktionalisierte Gruppe, die in der Lage ist, an ein festes Substrat zu binden, wobei die funktionalisierte Gruppe zumindest eine reaktive Silylgruppe umfasst;
eine photoaktivierbare Gruppe, die in der Lage ist, eine reaktive Gruppe bei Lichtexposition zu erzeugen, wobei die reaktive Gruppe in der Lage ist, eine screeningfähige Einheit zu binden; und
ein Polyethylenglykol-(PEG-)Polymer, das in der Lage ist, eine Monoschicht auf dem festen Substrat zu bilden, wobei die funktionalisierte Gruppe und die photoaktivierbare Gruppe an das Polymer angehaftet sind;
wobei das Polyethylenglykol ein Molekulargewicht im Bereich von 2000 g/mol bis 5000 g/mol aufweist,
wobei die Verbindung in der Lage ist, bei Kontakt mit dem festen Substrat eine Monoschicht am festen Substrat im Rahmen einer Ein-Schritt-Reaktion zu bilden, die mit der photoaktivierbaren Gruppe endet.

2. Verbindung nach Anspruch 1 mit der allgemeinen Formel I:
**X-L-Y** Formel I
wobei:
X die funktionalisierte Gruppe ist, die die zumindest eine reaktive Silylgruppe umfasst und in der Lage ist, an das feste Substrat zu binden;
L das PEG-Polymer ist, das in der Lage ist, die Monoschicht auf das feste Substrat zu bilden; und
Y die photoaktivierbare Gruppe ist, die in der Lage ist, die reaktive Gruppe bei der Lichtexposition zu erzeugern.

3. Verbindung nach einem der Ansprüche 1 und 2, wobei die reaktive Silylgruppe Trialkoxysilan ist.

4. Verbindung nach Anspruch 1, wobei das Polymer ein substituiertes oder unsubstituiertes Polyethylenglykol (PEG) umfasst.

5. Verbindung nach Anspruch 1, wobei die reaktive Gruppe, die bei der Lichtexposition der photoaktivierbaren Gruppe erzeugt wird, aus der Gruppe bestehend aus Amin, Hydroxy, Thiohydroxy, Halo, Alkoxy, Thioalkoxy, Aryloxy, Thioaryloxy, Carboxylat, Phosphat, Phosphonat, Sulfat und Sulfonat ausgewählt ist.

6. Verbindung nach Anspruch 1, wobei die photoaktivierbare Gruppe ein Carbamat umfasst.

7. Verbindung nach Anspruch 6, wobei die reaktive Gruppe, die bei der Lichtexposition der photoaktivierbaren Gruppe erzeugt wird, Amin ist.

8. Verbindung nach Anspruch 1, wobei die screeningfähige Einheit aus der Gruppe bestehend aus einer biologischen Einheit und einer chemischen Einheit ausgewählt ist.

9. Verfahren zum Herstellen einer Verbindung nach einem der Ansprüche 1 bis 8, wobei das Verfahren umfasst:
Bereitstellen eines Polyethylenglykol-(PEG-)Polymers mit einem Molekulargewicht im Bereich von 2000 g/mol bis 5000 g/mol, das eine erste reaktive Gruppe und eine zweite reaktive Gruppe enthält, wobei die erste reaktive Gruppe in der Lage ist, mit einer ersten Verbindung zu reagieren, wobei die erste Verbindung die funktionalisierte Gruppe enthält, die die zumindest eine Silylgruppe umfasst, und wobei die zweite reaktive Gruppe in der Lage ist, mit einer zweiten Verbindung zu reagieren, um dadurch die photoaktivierbare Gruppe zu bilden;
Reagieren des Polymers mit der ersten Verbindung; und
Reagieren des Polymers mit der zweiten Verbindung.

10. Verfahren nach Anspruch 9, wobei die erste Verbindung ferner eine dritte reaktive Gruppe umfasst, wobei die dritte reaktive Gruppe in der Lage ist, mit der ersten reaktiven Gruppe des PEG-Polymers zu reagieren.

11. Verfahren nach Anspruch 10, wobei die erste reaktive Gruppe und die dritte reaktive Gruppe eine Verknüpfungseinheit bilden, wobei die Verknüpfungseinheit die funktionalisierte Gruppe mit dem PEG-Polymer verknüpft.

12. Verfahren nach Anspruch 9, wobei die zweite Verbindung einen Rest der photoaktivierbaren Gruppe umfasst, der mit einer vierten reaktiven Gruppe verknüpft ist.

13. Verfahren nach Anspruch 12, wobei die vierte reaktive Gruppe in der Lage ist, mit der zweiten reaktiven Gruppe zu reagieren.

14. Verfahren nach Anspruch 9, wobei das Polymer ein ω-Amino-ω-carboxyl-PEG mit einem Molekulargewicht im Bereich von 2000 bis 5000 g/mol ist.

15. Verfahren zum Anhaften einer screeningfähigen Einheit an zumindest einem vorab ausgewählten Bereich eines festen Substrats, wobei das Verfahren umfasst:
Bereitstellen eines festen Substrats, wobei auf einer Oberfläche davon zumindest eine funktionelle Gruppe vorgesehen ist;
Bereitstellen einer Verbindung mit einer funktionalisierten Gruppe, die in der Lage ist, an das feste Substrat zu binden, wobei die funktionalisierte Gruppe zumindest eine reaktive Silylgruppe, eine photoaktivierbare Gruppe, die in der Lage ist, bei Exposition gegenüber einer Lichtquelle eine reaktive Gruppe zu erzeugen, wobei die reaktive Gruppe in der Lage ist, die screeningfähige Einheit zu binden, und ein Polyethylenglykol-(PEG-)Polymer, das in der Lage ist, eine Monoschicht am festen Substrat zu bilden und an dem die funktionalisierte Gruppe und die photoaktivierbare Gruppe angehaftet sind, umfasst, wobei das PEG-Polymer ein Molekulargewicht im Bereich von 2000 g/mol bis 5000 g/mol aufweist;
Inkontaktbringen der Verbindung mit dem festen Substrat im Rahmen einer Ein-Schritt-Reaktion durch Reagieren der funktionalisierten Gruppe mit der funktionellen Gruppe an der Oberfläche des festen Substrats, um damit ein festes Substrat bereitzustellen, an das eine Mehrzahl von Monoschichten des Polymers angehaftet ist, wobei jede der Monoschichten mit der photoaktivierbaren Gruppe endet;
Exponieren des zumindest einen vorab ausgewählten Bereichs des festen Substrats, an das die Monoschicht des Polymers angehaftet ist, gegenüber Licht, um dadurch die reaktive Gruppe aus der photoaktivierbaren Gruppe im vorab ausgewählten Bereich zu erzeugen; und
Binden der screeningfähigen Einheit an die reaktive Gruppe, wodurch die screeningfähige Einheit an den vorab ausgewählten Bereich des festen Substrats angehaftet wird.

16. Verfahren nach Anspruch 15, wobei das Binden der screeningfähigen Einheit an die reaktive Gruppe das Reagieren der reaktiven Gruppe mit der screeningfähigen Einheit umfasst.

17. Verfahren nach Anspruch 15, wobei das Binden der screeningfähigen Einheit an die reaktive Gruppe umfasst:
Bereitstellen einer Vermittlungseinheit, die in der Lage ist, an die screeningfähige Einheit und an die reaktive Gruppe zu binden; und
Binden der screeningfähigen Einheit an die reaktive Gruppe über die Vermittlungseinheit.

18. Verfahren nach Anspruch 17, wobei die Vermittlungseinheit ein Affinitätspaar umfasst

19. Verfahren nach Anspruch 17, wobei die Vermittlungseinheit ein Vermittlungsagens umfasst.

20. Verfahren zum Herstellen einer Reihe von screeningfähigen Einheiten, wobei das Verfahren umfasst:
Bereitstellen eines festen Substrats mit einer Mehrzahl an funktionellen Gruppen auf einer Oberfläche davon;
Bereitstellen einer Verbindung mit einer funktionalisierten Gruppe, die in der Lage ist, an das feste Substrat zu binden, wobei die funktionalisierte Gruppe zumindest eine reaktive Silylgruppe, eine photoaktivierbare Gruppe, die in der Lage ist, bei Exposition gegenüber einer Lichtquelle eine reaktive Gruppe, zu erzeugen, wobei die reaktive Gruppe in der Lage ist, eine screeningfähige Einheit zu binden; und ein Polyethylenglykol-(PEG-)Polymer, das in der Lage ist, eine Monoschicht am festen Substrat zu bilden und an dem die funktionalisierte Gruppe und die photoaktivierbare Gruppe angehaftet sind, umfasst, wobei das PEG-Polymer ein Molekulargewicht im Bereich von 2000 g/mol bis 5000 g/mol aufweist;
Inkontaktbringen der Verbindung mit dem festen Substrat durch Reagieren der funktionalisierten Gruppe mit den funktionellen Gruppen an der Oberfläche, um dadurch ein festes Substrat, an das eine Mehrzahl von Monoschichten des Polymers angehaftet sind, im Rahmen einer Ein-Schritt-Reaktion bereitzustellen, wobei jede der Monoschichten mit der photoaktivierbaren Gruppe endet;
Exponieren eines ersten vorab ausgewählten Bereichs des festen Substrats, an das die Monoschicht des Polymers angehaftet ist, gegenüber Licht, um dadurch eine reaktive Gruppe aus der photoaktivierbaren Gruppe im ersten vorab ausgewählten Bereich zu erzeugen; und
Binden einer screeningfähigen Einheit an die reaktive Gruppe im ersten vorab ausgewählten Bereich;
Exponieren eines zweiten vorab ausgewählten Bereichs des festen Substrats, an das die Monoschicht des Polymers angehaftet ist, gegenüber Licht, um dadurch eine reaktive Gruppe aus der photoaktivierbaren Gruppe im zweiten vorab ausgewählten Bereich zu erzeugen;
Binden einer zweiten screeningfähigen Einheit an die reaktive Gruppe im zweiten vorab ausgewählten Bereich,
Aufeinanderfolgendes Exponieren eines n-ten vorab ausgewählten Bereichs des festen Substrats, an das die Monoschicht des Polymers angehaftet ist, gegenüber Licht, um dadurch eine reaktive Gruppe im n-ten vorab ausgewählten Bereich zu erzeugen; und
Binden einer n-ten screeningfähigen Einheit an die reaktive Gruppe im n-ten vorab ausgewählten Bereich, um dadurch eine Reihe von screenningfähigen Einheiten zu bilden.

21. Verfahren nach Anspruch 20, wobei das Binden der screeningfähigen Einheit an die reaktive Gruppe umfasst:
Bereitstellen einer Vermittlungseinheit, die in der Lage ist, an die sweeningfähige Einheit und an die reaktive Gruppe zu binden; und
Binden der screeningfähigen Einheit an die reaktive Gruppe über die Vermittlungseinheit

22. Verfahren nach Anspruch 20, wobei der vorab ausgewählten Bereich des festen Substrats im Bereich zwischen 1 x 10⁻² cm' bis 1 x 10⁻² cm² und 1 x 10⁻⁵ bis 1 x 10⁻⁵ cm² liegt.

23. Verfahren nach Anspruch 20, wobei das n in einem Bereich von 2 bis 2000 liegt.

24. Reihe an screeningfähigen Einheiten, die gemäß dem Verfahren nach Anspruch 20 hergestellt sind.

25. Reihe an screeningfähigen Einheiten, umfassend:
ein festes Substrat, auf das eine Mehrzahl von Monoschichten eines Polyethylenglykol-(PEG)Polymers aufgetragen ist, wobei das PEG-Polymer ein Molekulargewicht im Bereich von 2000 g/mol bis 5000 g/mol aufweist und eine funktionalisierte Gruppe und eine photoaktivierbare Gruppe an dieses angehaftet sind, wobei die funktionalisierte Gruppe in der Lage ist, an das feste Substrat zu binden, und umfassend zumindest eine reaktive Silylgruppe, wobei die photoaktivierbare Gruppe in der Lage ist, eine reaktive Gruppe bei Exposition gegenüber einer Lichtquelle zu erzeugen, wobei die reaktive Gruppe in der Lage ist, die screeningfähige Einheit zu binden, wobei jede der Mehrzahl von Monoschichten des PEG-Polymers auf das feste Substrat im Rahmen einer Ein-Schritt-Reaktion durch Reagieren einer an das Polymer angehafteten funktionalisierten Gruppe mit einer funktionellen Gruppe auf einer Oberfläche des festen Substrats aufgetragen ist, wobei die funktionalisierte Gruppe zumindest eine reaktive Silylgruppe umfasst; und eine Mehrzahl von screeningfähigen Einheiten, die an die Mehrzahl von Monoschichten in vorab ausgewählten Bereichen des festen Substrats gebunden sind, so dass jede der screeningfähigen Einheiten an jede der Monoschichten in einem vorab ausgewählten Bereich des festen Substrats bei Lichtexposition einer Mehrzahl von Monoschichten des PEG-Polymers, wobei jede Monoschicht mit einer photoaktivierbaren Gruppe endet, im vorab ausgewählten Bereich des festen Substrats gebunden wird, wobei die Lichtexposition eine reaktive Gruppe erzeugt, die die sereeningfähige Einheit bindet.

26. Reihe nach Anspruch 25, wobei zumindest ein Teil der sereeningfähigen Einheiten direkt an die Mehrzahl von Monoschichten des PEG-Polymers gebunden ist.

27. Reihe nach Anspruch 25, wobei zumindest ein Teil der screeningfähigen Einheiten an eine Vermittlungseinheit gebunden ist, wobei die Vermittlungseinheit wiederum an die Mehrzahl von Monoschichten des PEG-Polymers gebunden ist.

28. Reihe nach Anspruch 25, wobei die Lichtexposition einer photoaktivierbaren Gruppe, die an die Monoschicht angehaftet ist, eine reaktive Gruppe im vorab ausgewählten Bereich des festen Substrats erzeugt.

29. Reihe nach Anspruch 25, wobei die screeningfähigen Einheiten aus der Gruppe bestehend aus biologischen Einheiten und chemischen Einheiten ausgewählt sind.

30. Anordnung nach Anspruch 25, wobei jeder der vorab ausgewählten Bereiche des festen Substrats im Bereich zwischen 1 x 10⁻² cm² bis 1 x 10⁻² cm² und 1 x 10⁻⁵ bis 1 x 10⁻⁵ cm² liegt.

31. Reihe nach Anspruch 25, die 1 bis 20.000 screeningfähige Einheiten umfasst.

32. Reihe nach Anspruch 25, wobei die Reihe durch ein Signal-RauschVerhältnis von zumindest 200 gekennzeichnet ist.

33. Reihe nach Anspruch 25, wobei die screeningfähigen Einheiten an das feste Substrat im Submikronbereich gebunden sind.

34. Festes Substrat, an das die Verbindung nach Anspruch 1 angehaftet ist.

## Revendications

1. Composé comprenant :
un groupe fonctionnalisé capable de se lier à un substrat solide, ledit groupe fonctionnalisé comprenant au moins un groupe silyle réactif ;
un groupe photo-activable capable de générer un groupe réactif quand il est exposé à une lumière, ledit groupe réactif étant capable de se lier à un fragment pouvant être criblé ; et
un polymère de polyéthylèneglycol (PEG) capable de former une monocouche sur ledit substrat solide, ledit polymère ayant ledit groupe fonctionnalisé et ledit groupe photo-activable rattachés à celui-ci ;
ledit polyéthylèneglycol ayant une masse moléculaire située dans la plage allant de 2000 g/mol à 5000 g/mol,
le composé étant capable de former, sur ledit substrat solide, une monocouche se terminant par ledit groupe photo-activable, lors de la mise en contact dudit substrat solide dans une réaction en une seule étape.

2. Composé selon la revendication 1, de formule générale I :
X-L-Y Formule I
dans laquelle :
X est ledit groupe fonctionnalisé comprenant ledit au moins un groupe silyle réactif et étant capable de se lier audit substrat solide ;
L est ledit polymère de PEG capable de former ladite monocouche sur ledit substrat solide ; et
Y est ledit groupe photo-activable capable de générer ledit groupe réactif quand il est exposé à ladite lumière.

3. Composé selon l'une quelconque des revendications 1 et 2, dans lequel ledit groupe silyle réactif est un trialcoxysilane.

4. Composé selon la revendication 1, dans lequel ledit polymère comprend un polyéthylèneglycol (PEG) substitué ou non substitué.

5. Composé selon la revendication 1, dans lequel ledit groupe réactif généré quand ledit groupe photo-activable est exposé à ladite lumière est choisi dans l'ensemble constitué par les groupes amine, hydroxy, thiohydroxy, halogéno, alcoxy, thioalcoxy, aryloxy, thioaryloxy, caxboxylate, phosphate, phosphonate, sulfate et sulfonate.

6. Composé selon la revendication 1, dans lequel ledit groupe photo-activable comprend un carbamate.

7. Composé selon la revendication 6, dans lequel ledit groupe réactif généré quand ledit groupe photo-activable est exposé à ladite lumière est un groupe amine.

8. Composé selon la revendication 1, dans lequel ledit fragment pouvant être criblé est choisi dans l'ensemble constitué par un fragment biologique et un fragment chimique.

9. Procédé pour préparer le composé de l'une quelconque des revendications 1 à 8, lequel procédé comprend les opérations consistant à :
disposer d'un polymère de polyéthylèneglycol (PEG) ayant une masse moléculaire située dans la plage allant de 2000 g/mol à 5000 g/mol et contenant un premier groupe réactif et un deuxième groupe réactif, ledit premier groupe réactif étant capable de réagir avec un premier composé, ledit premier composé contenant ledit groupe fonctionnalisé qui comprend ledit au moins un groupe silyle, et ledit deuxième groupe réactif étant capable de réagir avec un deuxième composé de façon à former ainsi ledit groupe photo-activable ;
faire réagir ledit polymère avec ledit premier composé ; et
faire réagir ledit polymère avec ledit deuxième composé.

10. Procédé selon la revendication 9, dans lequel ledit premier composé comprend en outre un troisième groupe réactif, ledit troisième groupe réactif étant capable de réagir avec ledit premier groupe réactif dudit polymère de PEG.

11. Procédé selon la revendication 10, dans lequel ledit premier groupe réactif et ledit troisième groupe réactif forment un fragment de liaison, ledit fragment de liaison liant ledit groupe fonctionnalisé audit polymère de PEG.

12. Procédé selon la revendication 9, dans lequel ledit deuxième composé comprend un résidu dudit groupe photo-activable qui est lié à un quatrième groupe réactif.

13. Procédé selon la revendication 12, dans lequel ledit quatrième groupe réactif est capable de réagir avec ledit deuxième groupe réactif.

14. Procédé selon la revendication 9, dans lequel ledit polymère est un ω-amino-ω-carboxyl-PEG ayant une masse moléculaire située dans la plage allant de 2000 à 5000 g/mol.

15. Procédé pour attacher un fragment pouvant être criblé à au moins une zone présélectionnée d'un substrat solide, lequel procédé comprend les opérations consistant à :
disposer d'un substrat solide ayant au moins un groupe fonctionnel sur une surface de celui-ci ;
disposer d'un composé ayant un groupe fonctionnalisé capable de se lier audit substrat solide, ledit groupe fonctionnalisé comprenant au moins un groupe silyle réactif, un groupe photo-activable capable de générer un groupe réactif quand il est exposé à une source de lumière, ledit groupe réactif étant capable de se lier audit fragment pouvant être criblé, et un polymère de polyéthylèneglycol (PEG) capable de former une monocouche sur ledit substrat solide et ayant ledit groupe fonctionnalisé et ledit groupe photo-activable rattachés à celui-ci, ledit polymère de PEG ayant une masse moléculaire située dans la plage allant de 2000 g/mol à 5000 g/mol ;
mettre ledit composé en contact avec ledit substrat solide, dans une réaction en une seule étape, par réaction dudit groupe fonctionnalisé avec ledit groupe fonctionnel sur ladite surface du substrat solide, de façon à former ainsi un substrat solide ayant une pluralité de monocouches dudit polymères rattachées à celui-ci, chacune desdites monocouches se terminant par ledit groupe photo-activable ;
exposer à une lumière ladite au moins une zone présélectionnée dudit substrat solide ayant ladite monocouche dudit polymère rattachée à celui-ci, de façon à générer ainsi ledit groupe réactif à partir dudit groupe photo-activable au niveau de ladite zone présélectionnée ; et
lier ledit fragment pouvant être criblé audit groupe réactif, en rattachant ainsi le fragment pouvant être criblé à la zone présélectionnée du support solide.

16. Procédé selon la revendication 15, dans lequel ladite liaison dudit fragment pouvant être criblé audit groupe réactif comprend la réaction dudit groupe réactif avec ledit fragment pouvant être criblé.

17. Procédé selon la revendication 15, dans lequel ladite liaison dudit fragment pouvant être criblé audit groupe réactif comprend les opérations consistant à :
disposer d'un fragment de médiation capable de se lier audit fragment pouvant être criblé et audit groupe réactif ; et
lier ledit fragment pouvant être criblé audit groupe réactif via ledit fragment de médiation.

18. Procédé selon la revendication 17, dans lequel ledit fragment de médiation comprend une paire d'affinité.

19. Procédé selon la revendication 17, dans lequel ledit fragment de médiation comprend un agent de médiation.

20. Procédé pour préparer un réseau de fragments pouvant être criblés, lequel procédé comprend les opérations consistant à :
disposer d'un substrat solide ayant une pluralité de groupes fonctionnels sur une surface de celui-ci ;
disposer d'un composé ayant un groupe fonctionnalisé capable de se lier audit substrat solide, ledit groupe fonctionnalisé comprenant au moins un groupe silyle réactif, un groupe photo-activable capable de générer un groupe réactif quand il est exposé à une source de lumière, ledit groupe réactif étant capable de lier un fragment pouvant être criblé ; et un polymère de polyéthylèneglycol (PEG) capable de former une monocouche sur ledit substrat solide et ayant ledit groupe fonctionnalisé et ledit groupe photo-activable rattachés à celui-ci, ledit polymère de PEG ayant une masse moléculaire située dans la plage allant de 2000 g/mol à 5000 g/mol
mettre ledit composé en contact avec ledit substrat solide par réaction dudit groupe fonctionnalisé avec lesdits groupes fonctionnels sur ladite surface, de façon à former ainsi, dans une réaction en une seule étape, un substrat solide ayant une pluralité de monocouches dudit polymère rattachées à celui-ci, chacune desdites monocouche se terminant par ledit groupe photo-activable ;
exposer à une lumière une première zone présélectionnée dudit substrat bolide ayant ladite monocouche dudit polymère rattachée à celui-ci, de façon à générer ainsi un groupe réactif à partir dudit groupe photo-activable au niveau de ladite première zone présélectionnée ; et
lier un fragment pouvant être criblé audit groupe réactif au niveau de ladite première zone présélectionnée ;
exposer à une lumière une deuxième zone présélectionnée dudit substrat solide ayant ladite monocouche dudit polymère rattachée à celui-ci, de façon à générer ainsi un groupe réactif à partir dudit groupe photo-activable au niveau de ladite deuxième zone présélectionnée ;
lier un deuxième fragment pouvant être criblé audit groupe réactif au niveau de ladite deuxième zone présélectionnée ;
exposer consécutivement à une lumière une Nième zone présélectionnée dudit substrat solide ayant ladite monocouche dudit polymère rattachée à celui-ci, de façon à générer ainsi un groupe réactif au niveau de ladite Nième zone présélectionnée ; et
lier un Nième fragment pouvant être criblé audit groupe réactif au niveau de ladite Nième zone présélectionnée, en formant ainsi un réseau de fragments pouvant être criblés.

21. Procédé selon la revendication 20, dans lequel ladite liaison dudit fragment pouvant être criblé audit groupe réactif comprend les opérations consistant à :
disposer d'un fragment de médiation capable de se lier audit fragment pouvant être criblé et audit groupe réactif ; et
lier ledit fragment pouvant être criblé audit groupe réactif via ledit fragment de médiation.

22. Procédé selon la revendication 20, dans lequel ladite zone présélectionnée dudit substrat solide est située dans la plage comprise entre 1 x 10⁻² cm² pour 1 x 10⁻² cm² et 1 x 10⁻⁵ cm² pour 1 x 10⁻⁵ cm².

23. Procédé selon la revendication 20, dans lequel ledit N est situé dans la plage allant de 2 à 2000.

24. Réseau de fragments pouvant être criblés, préparé par le procédé de la revendication 20.

25. Réseau de fragments pouvant être criblés, comprenant un substrat solide ayant une pluralité de monocouches d'un polymère de polyéthylèneglycol (PEG) appliquées sur celui-ci, ledit polymère de PEG ayant une masse moléculaire située dans la plage allant de 2000 g/mol à 5000 g/mol et ayant un groupe fonctionnalisé et un groupe photo-activable rattachés à celui-ci, ledit groupe fonctionnalisé étant capable de se lier audit substrat solide, et comprenant au moins un groupe silyle réactif, ledit groupe photo-activable étant capable de générer un groupe réactif quand il est exposé à une source de lumière, ledit groupe réactif étant capable de se lier audit fragment pouvant être criblé, dans lequel chacune parmi ladite pluralité de monocouches dudit polymère de PEG est appliquée sur ledit substrat solide, dans une réaction en une seule étape, par réaction d'un groupe fonctionnalisé rattaché audit polymère avec un groupe fonctionnel sur une surface dudit substrat solide, ledit groupe fonctionnalisé comprenant au moins un groupe silyle réactif ; et une pluralité de fragments pouvant être criblés qui sont liés à ladite pluralité de monocouches au niveau de zones présélectionnées dudit substrat solide, de façon que chacun desdits fragments pouvant être criblés soit lié à chacune desdites monocouches au niveau d'une zone présélectionnée dudit substrat solide après exposition à la lumière d'une pluralité de monocouches dudit polymère de PEG où chaque monocouche se termine par un groupe photo-activable, au niveau de ladite zone présélectionnée dudit substrat solide, et dans lequel ladite exposition à la lumière génère un groupe réactif qui se lie audit fragment pouvant être criblé.

26. Réseau selon la revendication 25, dans lequel au moins une portion desdits fragments pouvant être criblés est liée directement à ladite pluralité de monocouches dudit polymère de PEG.

27. Réseau selon la revendication 25, dans lequel au moins une portion desdits fragments pouvant être criblés est liée à un fragment de médiation, tandis que ledit fragment de médiation est lié à ladite pluralité de monocouches dudit polymère de PEG.

28. Réseau selon la revendication 25, dans lequel ladite exposition à la lumière d'un groupe photo-activable qui est rattaché à ladite monocouche génère un groupe réactif au niveau de ladite zone présélectionnée dudit substrat solide.

29. Réseau selon la revendication 25, dans lequel lesdits fragments pouvant être criblés sont choisis dans l'ensemble constitué par les fragments biologiques et les fragments chimiques.

30. Réseau selon la revendication 25, dans lequel chacune desdites zones présélectionnées dudit substrat solide est située dans la plage comprise entre 1 x 10⁻² cm² pour 1 x 10⁻² cm² et 1 x 10⁻⁵ cm² pour 1 x 10⁻⁵ cm².

31. Réseau selon la revendication 25, comprenant de 1 à 20 000 fragments pouvant être criblés.

32. Réseau selon la revendication 25, lequel réseau est **caractérisé par** un rapport signal/bruit d'au moins 200.

33. Réseau selon la revendication 25, dans lequel lesdits fragments pouvant être criblés sont liés audit substrat solide avec une résolution sous-micrométrique.

34. Substrat solide sur lequel est rattaché le composé de la revendication 1.
